(19) Europäisches Patentamt
European Patent Office
Office européen des brevets

(11) Publication number: **0 257 921 B1**

(12) **EUROPEAN PATENT SPECIFICATION**

(45) Date of publication of patent specification: **15.04.92**

(51) Int. Cl.5: **C07C 43/21**, C07C 43/215, C07C 43/225, C07C 69/708, C07C 69/157, C07C 235/02, C07C 323/00, C07C 323/04, C07C 323/13, C07C 317/00, C07C 317/12

(21) Application number: **87307133.6**

(22) Date of filing: **12.08.87**

(54) **New 1,3-diaryl cyclopentanes and derivatives thereof as paf antagonists.**

(30) Priority: **21.08.86 US 898966**

(43) Date of publication of application:
**02.03.88 Bulletin 88/09**

(45) Publication of the grant of the patent:
**15.04.92 Bulletin 92/16**

(84) Designated Contracting States:
**CH DE FR GB IT LI NL**

(56) References cited:
**EP-A- 0 144 804**

**CHEMICAL ABSTRACTS, vol. 40, no. 12, 1946, columns 3408-8 - 3409-1, Columbus, Ohio, USA; P. RUGGLI et al, "Ketones, ketonic acids and enol lactones. IX. Cyclopentane-1,3-diketones"**

(73) Proprietor: **MERCK & CO. INC.**
**126, East Lincoln Avenue P.O. Box 2000**
**Rahway New Jersey 07065-0900(US)**

(72) Inventor: **Chiang, Yuan-Ching P.**
**73 Mitchell Avenue**
**Piscataway New Jersey 08854(US)**
Inventor: **Biftu, Tesfaye**
**25 Victorian Drive**
**Old Bridge New Jersey 08859(US)**
Inventor: **Graham, Donald W.**
**294 Old Tote Road**
**Mountainside New Jersey 07092(US)**
Inventor: **Yang, Shu Shu**
**324 Eileen Way**
**Bridgewater New Jersey 08807(US)**
Inventor: **Chang, Michael N.**
**746 Austin Street**
**Westfield New Jersey 07090(US)**
Inventor: **Chabala, John C.**
**560 Sherwod Parkway**
**Westfield New Jersey 07090(US)**

**EP 0 257 921 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Inventor: **Thomson, Kathryn L.**
**312 Washington Street**
**Westfield New Jersey 07090(US)**

(74) Representative: **Hesketh, Alan, Dr.**
**European Patent Department Merck & Co.,**
**Inc. Terlings Park Eastwick Road**
**Harlow Essex, CM20 2QR(GB)**

## Description

Platelet-activating factor (PAF) has recently been identified as an acetyl glyceryl ether phosphorylcholine (AGEPC), i.e., 1-O-hexadecyl/ octadecyl-2-acetyl-sn-glyceryl-3-phosphorylcholine (Hanahan D.J., et al., J. Biol. Chem. 255:5514, 1980). Even before its chemical identification, PAF had been linked to various biological activities and pathways making it one of the important mediators responsible for a variety of physiological processes including activation or coagulation of platelets, pathogenesis of immune complex deposition, smooth muscle contraction, inflammation, hypotension, shock, pain, edema as-well as respiratory, cardiovascular, renal and intravascular alterations. Since these physiological processes are in turn associated with a large group of diseases, for example, inflammatory disease, nephritis, stroke and other cardiovascular disorders, hypotension, shock, psoriasis, allergic and skin diseases, asthma, lung edema, peptic or stomach ulcer, dental pain, and adult respiratory distress syndrome, more and more scientific investigation has been focused on the search of a PAF antagonist or inhibitor for treating or preventing these common diseases.

EP-A-0144804 describes a class of 2,5-diaryl tetrahydrofuran derivatives which are stated to have potent and specific PAF antagonist activities and thus to be useful in the treatment of various diseases or disorders mediated by PAF.

The compounds of the present invention are specific PAF antagonists which can exist in various isomers. For example, the cyclopentane derivatives of formula (A) can exist in four different stereoisomers as shown in Scheme I. Each stereoisomers in turn exists in a pair of $\pm$ enantiomers.

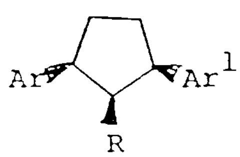

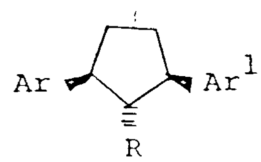

(A)

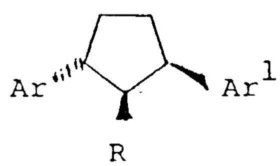

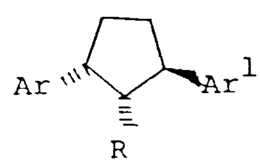

Scheme I

These isomers bear a close relationship to the PAF antagonistic activity observed for the compounds within the scope of this invention.

Accordingly, it is the object of the present invention to prepare the most potent isomers of known or novel cyclopentane derivatives as PAF antagonists and use them for the treatment of various diseases including prevention of platelet aggregation, hypotension, inflammation, asthma, lung edema, adult respiratory distress syndrome, various shock syndromes, cardiovascular disorders and other related skeletal-muscular disorders.

Another object of the present invention is to develop processes for the preparation of the 1,3-diarylcyclopentane derivatives.

A further object of the present invention is to provide acceptable pharmaceutical compositions containing one or more of the cyclopentane derivatives as the active ingredient. As PAF antagonists, these novel compositions should be effective in the treatment of various PAF-induced diseases.

Finally, it is the object of this invention to provide a method of treatment comprising the administration of a therapeutically sufficient amount of these PAF antagonists to a patient suffering from various disorders including inflammation, e.g., osteoarthritis, rheumatoid arthritis, gout, and nephritis, hypotension, shock, psoriasis, allergic or skin diseases, asthma, pain especially dental pain, peptic or stomach ulcer, lung edema, adult respiratory distress syndrome or stroke and other cardiovascular disorders.

DETAILED DESCRIPTION OF THE INVENTION

A. Scope of the Invention

This invention relates to PAF antagonists of the structural formula (I)

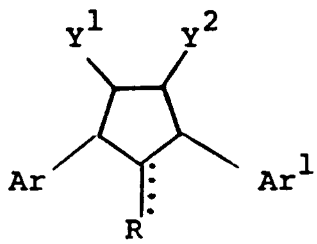

(I)

wherein

R is (a) when R is connected to the cyclopentane ring by a single bond:

(1) $-(X)_m(CH_2)_nR^1$ wherein X is O, $NR^2$, $N^+R^2R^3$, S, SO, $SO_2$, $SO_3$ or CO; m is 0 or 1; n is 1-6; $R^1$, $R^2$, $R^3$ are as defined below;

(2) $-X^1R^{10}$ wherein $X^1$ is O, S, $NR^2$ or $N^+R^2R^3$ and $R^{10}$ is $R^1$ except that it cannot be definitions (h)-(r) of $R^1$;

(3) $-X^2R^2$ wherein $X^2$ is SO, $SO_2$, $SO_3$, $SO_2NR^3$, $NR^3(CO)$, $NR^3(CO)NH$, or $NHSO_2$; or

(4) $R^1$;

(b) when R is connected to the cyclopentane ring by a double bond:

(1) $=C-R^2$;

(2) $=O$; or

(3) $=NOR^2$;

$R^1$ is (a) H;

(b) loweralkyl especially $C_{1-8}$ alkyl; e.g. methyl, ethyl, isopropyl, n-propyl, n-butyl, t-butyl, pentyl, hexyl, heptyl or octyl;

(c) loweralkenyl especially $C_{2-8}$ alkenyl, e.g. vinyl, allyl, $CH_3CH=CHCH_2CH_2$-, $CH_3(CH_2)_3CH=CH$- and $(CH_3)_2C=CH$-;

(d) lowercycloalkyl especially $C_{3-8}$ cycloalkyl;

(e) loweralkynyl especially $C_{2-8}$ alkynyl, e.g., $-C\equiv CH$;

(f) $-(CO)NR^2R^3$ wherein $R^2$ and $R^3$ independently are H, $C_{1-8}$ alkyl, $C_{3-8}$ cycloalkyl, e.g., cyclopropyl, cyclopentyl and cyclohexyl, or $C_{1-4}$ perhaloalkyl such as $CF_3$-;

(g) $-(CO)R^2$;

(h) $-S(O)_qR^2$ where q represents 0-2;

(i) $-O(CO)R^2$;

(j) $-SO_2NR^2R^3$;

(k) $-NR^2(CO)R^3$;

(l) $-NR^2(CO)NHR^3$;

(m) $-NHSO_2R^2$;

(n) $-OR^2$

(o) $-NR^2R^3$;

(p) -CN;

(q) $-NO_2$

(r) halo e.g., Cl, F, Br and I;

(s) perhaloloweralkyl, e.g., $-CF_3$;

(t) phenyl or substituted phenyl as defined below; or

(u) $-(CO)OR^2$;

Ar and $Ar^1$ are the same or different from each other and are

(a) phenyl or substituted phenyl of formula

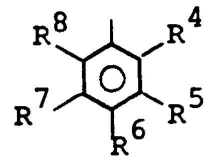

where $R^4$-$R^8$ independently represent H; $R^2$; YO- wherein Y is $R^2$, loweralkenyl such as $-CH_2-CH=CH_2$; loweralkynyl such as -CH-C≡CH,

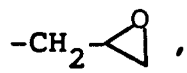

$-CH_2-C(O)OR^2$, $-CH_2-OR^2$, $-CH_2C_{3\text{-}8}$-cycloalkyl such as

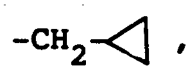

$-CH_2AR^2$ wherein $Ar^2$ is phenyl or substituted phenyl, $-CH_2-CH(OH)-CH_2OH$; $R^2S$-; $R^2SO$; $R^2SO_2$; $CF_3O$-; $CF_3S$-; $CF_3SO$; $CF_3SO_2$; $CH_3OCH_2$-O-; $R^2R^3N$-; $OCH_2CO_2R^2$; $-SO_2NR^2R^3$; $-CO_2R^2$; $-CONR^2R^3$; -$NR^2COR^3$; $-OCONH_2$; $-CR^2R^3R^9$ wherein $R^9$ is the same or different from $R^2$; $-CH_2OR^2$; $-CH_2CO_2R^2$; $-CH_2OCOR^3$; $-CH_2O$-CO-$OR^2$; $-NHCH_2COOR^2$; halo such as F, Cl, Br and I; $N^+R^2R^3R^9W^-$ wherein $W^-$ is an anion such as $OH^-$, $Cl^-$, $Br^-$ or $I^-$; $NR^2SO_2R^3$; $COR^2$; $NO_2$; CN; or $R^4$-$R^5$. $R^5$-$R^6$, $R^6$-$R^7$ and $R^7$-$R^8$ joined together and form a ring, for example, $-OCH_2O$-, $-OCH_2CH_2$-O- or $-OCH_2CH_2N$-. The representative phenyl groups can be 3-methoxy-4-methylthiophenyl, 4-trifluoromethoxyphenyl, 3-methoxy-4-trifluoromethoxyphenyl, 3,4-dimethoxyphenyl, 3-methoxy-4-dimethylaminophenyl, and 3,4,5-trimethoxyphenyl;

(b) monoheteroaryl, di- or polyheteroaryl, or fused heteroaryl containing from 1 to 3 of any one or more of the heteroatoms N, S or O in each heteroaryl ring thereof and each ring may either be unsubstituted or substituted appropriately with a radical selected from a group consisting of $R^4$-$R^8$, for example, pyridyl, pyrryl, thienyl, isothiazolyl, imidazolyl, tetrazolyl, pyrazinyl, pyrimidyl, quinolyl, isoquinolyl, benzothienyl, isobenzofuryl, pyrazolyl, indolyl, purinyl, carbozolyl, and isoxazolyl and the like.

The following are preferred heteroaryl groups:

(1) pyrryl or pyrryl substituted with $R^4$-$R^6$;
(2) furyl or furyl substituted with $R^4$-$R^6$;
(3) pyridyl or pyridyl substituted with $R^4$-$R^7$;
(4) thiophene or thiophene substituted with $R^4$-$R^6$;
(5) thiazolyl or thiazolyl substituted with $R^4$-$R^5$; or
(6) pyrimidyl or pyrimidyl substituted with $R^4$-$R^6$; and

$R^1$ and $Y^2$ independently are H; $C_{1\text{-}6}$ alkyl, especially $CH_3$; F or chloro; or $C_{1\text{-}6}$ haloalkyl especially $CF_3$.

Preferably, the PAF antagonists of this invention are of structural formula

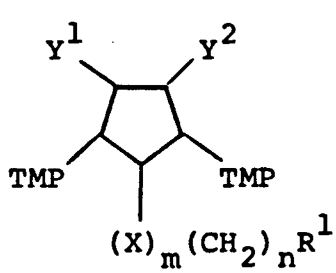

or an isomer thereof wherein TMP is 3,4,5-trimethoxyphenyl; X is O or S; m is 0-1, n is 1-2; $Y^1$, $Y^2$ and $R^1$ are as previously defined.

Another preferred mode of this invention involves compounds of structural formula

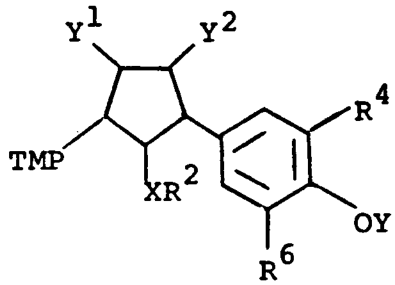

or an isomer thereof wherein TMP, $R^4$, $R^6$, Y, $Y^1$, $Y^2$ and $R^2$ are as previously defined; X is O or S.

Still another preferred mode of this invention involves compounds of formula

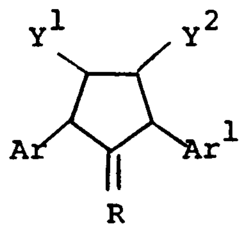

wherein Ar is as previously defined; R is $NOR^2$ or O; and $Ar^1$ is TMP or

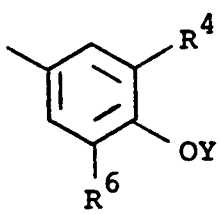

where $R^4$, Y, $Y^1$, $Y^2$ and $R^6$ are as previously defined.

B. Preparation of the compounds within the scope of the invention.

The PAF antagonists of this invention have been prepared largely by reductions of appropriately substituted cyclopentanones or their corresponding hydrazone derivatives:

Scheme (a)

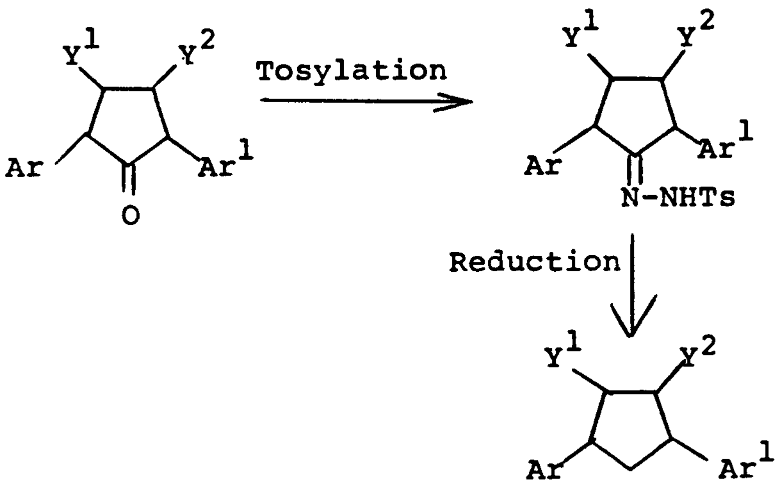


Scheme (b)

Scheme (c)

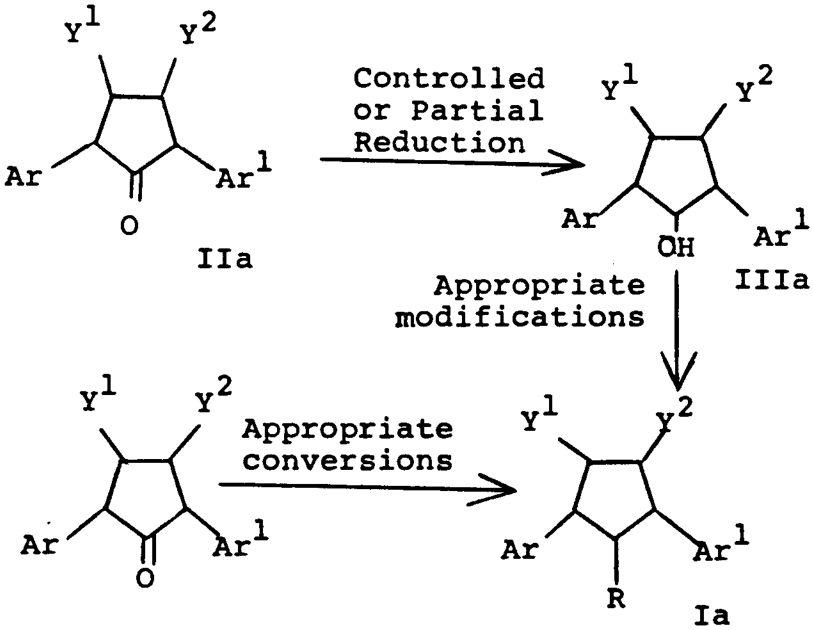


IIa

IIIa

Ia

According to Scheme (a), the cyclopentanone derivative II is first converted to the tosylhydrazone derivative IIb which in turn is reduced under mild temperatures (about 50 °C to 150 °C) by $NaBH_3CN$ in dimethylformamide (DMF) and sulfolane in the presence of tosyl acid. In addition to $NaBH_3CN$, other commercially available reducing agents such as $NaBH_4$, hydrazine ($NH_2NH_2$) with KOH or t-Buok and Zn with $NH_3$ in an aprotic polar solvent, e.g., dioxane, diethylene glycol, dimethylsulfoxide, alcohol, tetrahydrofuran and the like may also be used.

In Scheme (b), a cyclopentanone derivative II is reduced by $LiAlH_4$, $NaBH_4$, $LiBH_4$, $NaAlH_2$-$(CH_3OCH_2CH_2OCH_3)_2$ under a controlled manner to a cyclopentanol derivative IIa followed by appropriate modifications to a compound of Formula Ia. One of such modifications is alkylation with a reagent of Formula $R^2X$ wherein X is halo, tosyl or mesyl in an aprotic solvent (e.g., DMSO, HMPA, THF, $CH_3OCH_2CH_2OCH_3$, xylene and toluene) and at about 25° to 150 °C in the presence of a base such as $Na^+DMSO^-$, $NaNH_2$, $Ag_2O$, n-BuLi and $KN[Si(CH_3)_3]_2$.

According to Scheme (c), the cyclopentanone derivative IIa may also be converted directly via appropriate conversions, for example, acetylation as described below in Scheme (d) and amination as described in Scheme (e).

Scheme (d)

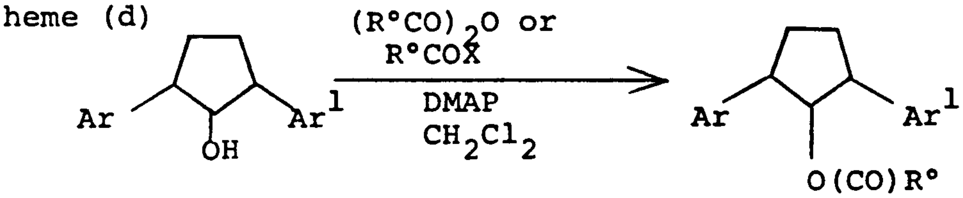


wherein X is halo;
R° is $C_{1-6}$alkyl; and
DMAP is 4-dimethylaminopyridine

Scheme (e)

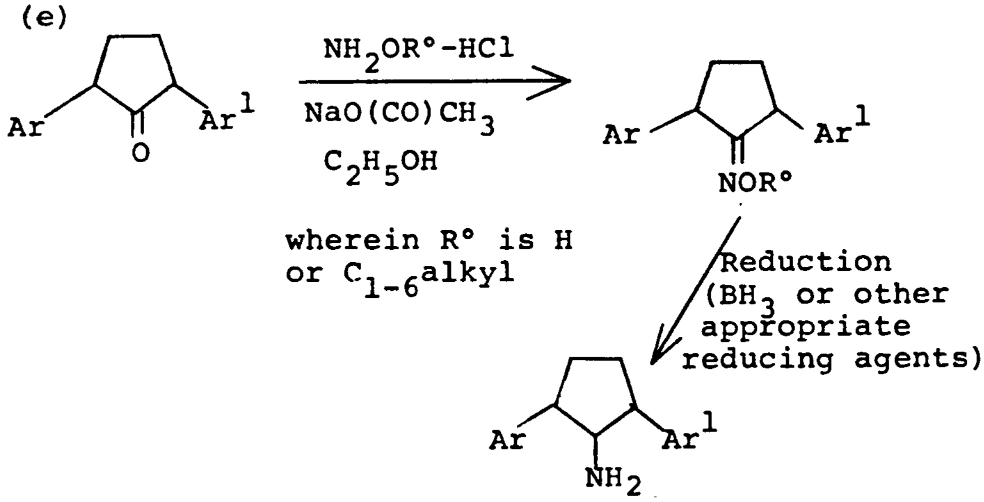


The conversion of one cyclopentane derivative to the other can be illustrated by the following examples:

Scheme (f)

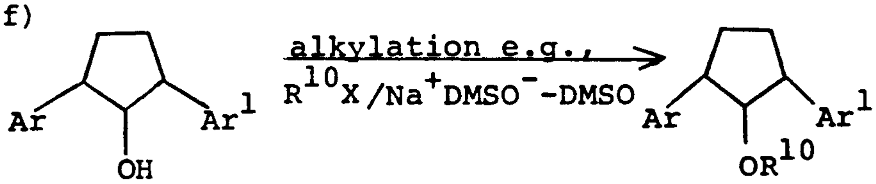


wherein $R^{10}$ is $R^2$; $-CH_2COOR°$; $-CH_2-C≡CH$; $CH_2C≡N$; $-CH_2CH=CH_2$; $-CH_2$-phenyl or $-CH_2$-substituted phenyl as previously defined; $-(CH_2)_2OC_{1-6}$ alkyl; $-(CH_2)_2NC_{1-6}$alkyl; or $-CH_2CONR^2R^3$.

Scheme (g)

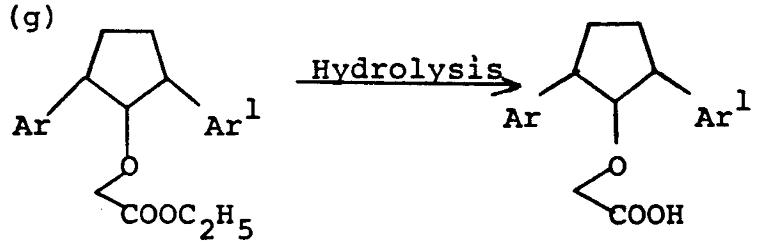


OR

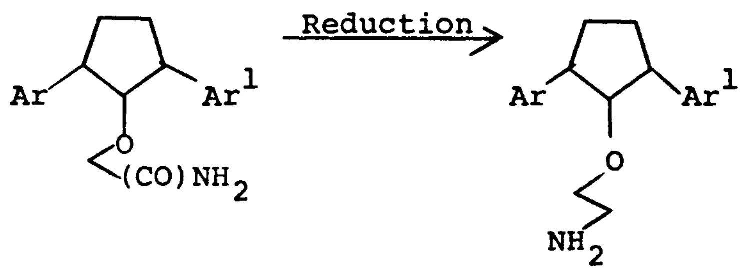


Scheme (h)

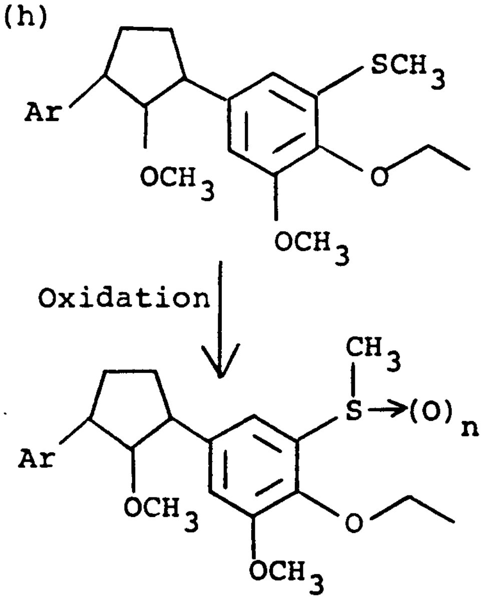


wherein n is 1 or 2

OR

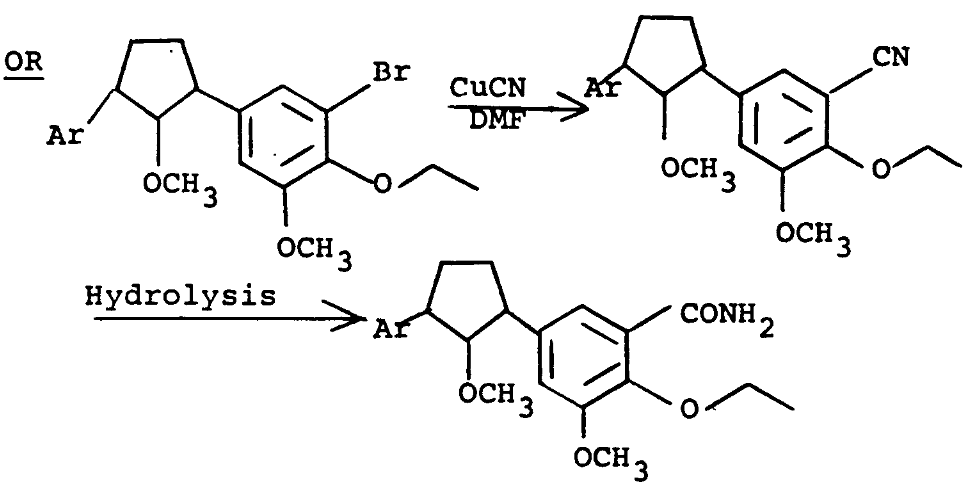


The cyclopentanone starting material (II) can be prepared readily according to the following procedures, for example:

Method A: Preparation of Symmetrical Cyclopentanone Derivatives:

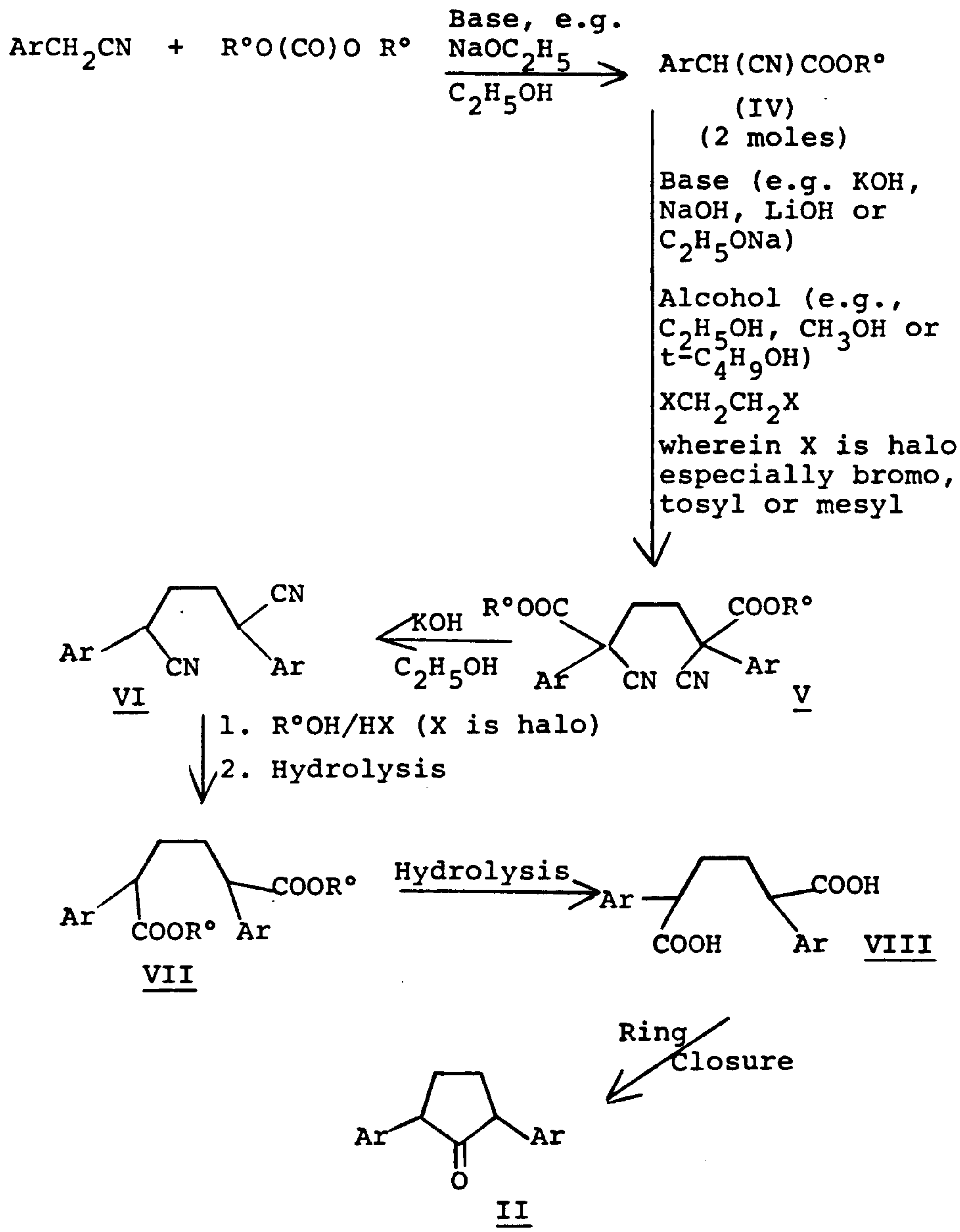


The formation of Compound IV and Compound V can also be effected by other commonly available bases, for example, $KOC_2H_5$, $NaOCH_3$, NaH, KH, $NaNH_2$ and t-Buok. Various solvent systems may be used, for example, xylene, benzene, $(C_2H_5)_2O$, DMF, DMSO, $CH_3O\ CH_2CH_2OCH_3$, THF or an alcohol such as methanol or ethanol. The reaction is normally conducted under mild temperatures from about 0°C to about 150°C, preferably, from 25°C to 100°C.

The conversion of Compound V to VI can be affected by a strong base including KOH, LiOH and NaOH. The reaction is normally carried out at about 0°C to about 150°C preferably 25°C-100°C in a protic solvent such as methanol and ethanol.

The conversion of Compound VI to VII can be efffected by a strong acid such as HCl, HBr, HI, TsOH, $H_2SO_4$ or $H_3PO_4$ in an alcohol such as ethanol or methanol at about 20°C-100°C preferably 0°C-40°C followed by hydrolysis to VII. Compound VII in turn can be hydrolyzed to Compound VIII under conventional procedures. The catalysts for the hydrolysis can be acidic such as HCl, HBr and the like or basic such as KOH, NaOH or LiOH.

In order to facilitate ring closure of VIII which is normally conducted for about 2-10 minutes, preferably less than 5 minutes at high temperatures up to 290 °C, preferably no higher than 270 °C in the presence of acetic anhydride or other dehydrating agent, e.g., $CH_3COX$, $C_2H_5O(CO)X$, DCC, and $(CF_3CO)_2O$. Alternatively the diacid VII can be converted to heavy metal salts such as those of Fe, Pb, Cd and Tl or alkaline earth metal salts such as those of Ba and Ca which in turn are pyrolyzed to give the symmetric cyclopentanones of Formula II.

Method B: Synthesis of Asymmetric Cyclopentanone Derivatives

As exemplified by Example 2, the synthesis of an asymmetric cyclopentanone involves the preparation of $ArC(CN)(COOR°)CH_2CH_2Br$ (VII) and $Ar^1CH(CN)COOR°$ according to the following scheme: wherein Ar and $Ar^1$ are not identical.

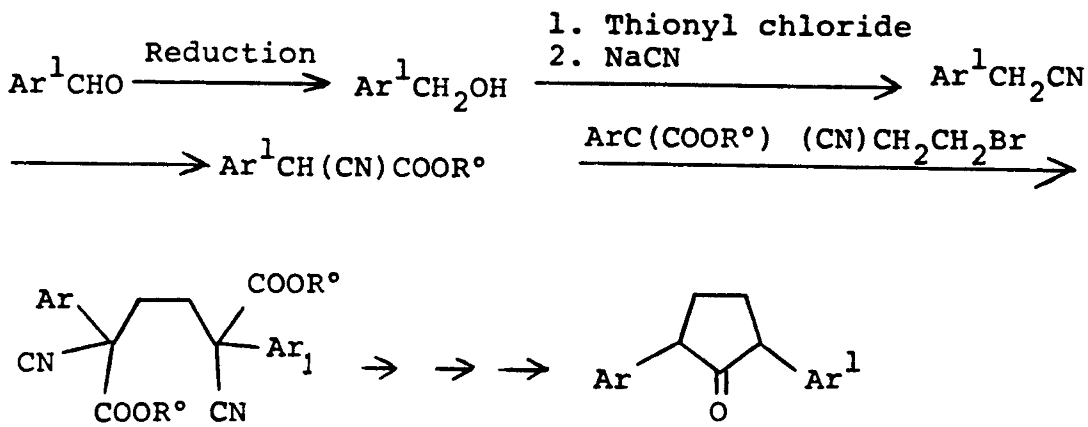


The preparation of $ArC(CN)(COOR°)CH_2CH_2X$ requires the reaction between $ArCH(CN)COOR°$ and an excess amount of $XCH_2CH_2X$ wherein X is halo preferably Br:

$ArCH(CN)COOR° + XCH_2CH_2X \dashrightarrow ArC(CN)COOR°)CH_2CH_2X$

The following examples illustrate how to prepare the compounds of the present invention.

EXAMPLE 1

**1α-Methoxy-2α,5β-bis-(3,4,5-trimethoxyphenyl)cyclopentane and ether analogs**

Step A:

**Preparation of α,α'-bis(3,4,5-Trimethoxyphenyl)adiponitrile**

Most of the ethanol was removed by distillation at reduced pressure (bath <90 °C) from a solution of 17.3 g (0.752 g-atom) of sodium in ethanol (377 ml). 3,4,5-Trimethoxyphenylacetonitrile (150g, 0.725 moles), diethyl carbonate (430 ml), and toluene (300 ml) were added, and the mixture heated under reflux in a nitrogen atmosphere with stirring. Ethanol was distilled while toluene was added at the same rate from a dropping funnel. When the distillation temperature reached 110 °, the slurry was cooled and filtered. The yellow solid was washed with toluene and ether, dried briefly in a vacuum oven and suspended in ethanol (315 ml) and 1,2-dibromoethane (27.1 ml, 0.315 mole). The slurry was heated under reflux in a nitrogen atmosphere with stirring for 7 hours. After standing for 7 hours at room temperature the reaction mixture was transferred using 200 ml of ethanol into a solution of potassium hydroxide (61.9 g, 85.6%, 0.945 moles) in 2000 ml of ethanol. The suspension was heated at 50-70 °C with stirring for 1.5 hours. After cooling the brown solid was filtered, washed with water (four times) and dried to give 76.8 g (48%) of α,α'-bis-(3,4,5-trimethoxyphenyl)adiponitrile, m.p. 173-174.5 °.

Step B:

**Preparation of Dimethyl α,α′-bis(3,4,5-trimethoxyphenyl)adipate**

α,α′-bis(3,4,5-Trimethoxyphenyl)adiponitrile (11 g, 25 mmoles) was suspended in methanol (100 ml) and a vigorous stream of hydrogen chloride gas was passed into the slurry for 30 minutes while the temperature was kept below 30°C with an ice bath. The pale yellow slurry was stirred at room temperature for 5.5 hours during which time the dinitrile slowly dissolves, then the iminoester hydrochloride precipitates suddenly. Ether (100 ml) was added and the solid quickly filtered, washed with ether and sucked dry briefly. The damp solid was immediately dissolved in water (100 ml). After a few minutes a solid precipitated. After stirring for 19 hours at room temperature the colorless solid was filtered, washed with water (three times) and dried to give 11.0 g (87%) of dimethyl α,α′-bis(3,4,5-trimethoxyphenyl)adipate, m.p. 179-183.5°.

Step C:

**Preparation of α,α′-bis(3,4,5-trimethoxyphenyl)adipic acid**

A mixture of dimethyl α,α′-bis(3,4,5-trimethoxyphenyl)adipate (11.0 g, 21.7 mmoles), potassium hydroxide (13.0 g, 202 mmoles), water (163 ml), and ethanol (65 ml) was heated at 90° in a nitrogen atmosphere for 3 hours. After cooling the clear solution was added slowly to 250 ml of 1N hydrochloric acid with vigorous stirring. The slurry was stirred and warmed to 50° for several minutes. After cooling in an ice bath the solid was filtered, washed with water (4 times) and dried to give 10.07 g (97%) of α,α′-bis(3,4,5-trimethoxyphenyl)adipic acid, m.p. 226-233°. NMR (DMSO-$d_6$) indicates a 9:1 mixture of meso and racemic isomers.

Step D:

Preparation of trans-2,5-bis(3,4,5-Trimethoxyphenyl)cyclopentanone

A mixture of α,α′-bis(3,4,5-trimethoxyphenyl)adipic acid (10 g, 21 mmoles) and acetic anhydride (30 ml) was heated at 150°C for 30 minutes. Acetic acid and anhydride were removed in vacuo. The flask containing the solid residue was fitted with a distillation head and while under vacuum (~150 mm) was placed in an oil bath heated to 287°C. The solid melted; there was a vigorous evolution of gas and about 5 ml of liquid distilled. After 6.5 minutes the flask was cooled rapidly with a water bath. The yellow-brown syrupy residue was dissolved in 80 ml of hot ethanol, filtered from some insoluble material and seeded. After standing at room temperature overnight, the crystalline solid was filtered, washed with cold ethanol (3 times) and dried to give 5.57 g (64%) of trans-2,5-bis(3,4,5-trimethoxyphenyl)cyclopentanone, m.p. 140-142.5°. IR 1740$cm^{-1}$ (C=0), mass spec m/e 416 ($M^+$).

Step E:

**Preparation of Trans-2,5-bis(3,4,5-trimethoxyphenyl)cyclopentanol**

To a suspension of 2,5-bis(3,4,5-trimethoxyphenyl)cyclopentanone (1.16 g, 2.79 mmoles) in THF (15 ml) in a nitrogen atmosphere and cooled in an ice bath was added all at once lithium aluminum hydride (0.106 g, 2.79 mmoles). The cooling bath was removed, and the suspension stirred at room temperature for 3 hours. The mixture was cooled in an ice bath, and $H_2O$ (106 μl) was added cautiously followed by addition of 106 μl of 15% NaOH and 318 μl of $H_2O$. The suspension was stirred at room temperature for 30 minutes, $CH_2Cl_2$ (20 ml) was added, and the solid filtered and washed with $CH_2Cl_2$ (three times). The combined filtrate and washes were evaporated in vacuo. The syrupy residue (1.18 g) was crystallized from $CH_2Cl_2$-hexane to give 1.06 g (91%) of trans-2,5-bis(3,4,5-trimethoxyphenyl)cyclopentanol, m.p. 126-128°.

Step F:

Preparation of 1α-Methoxy-2α,5β-bis-(3,4,5-trimethoxyphenyl)cyclopentane

To a solution of trans-2,5-bis(3,4,5-trimethoxyphenyl)cyclopentanol (125 mg, 0.3 mmole) in DMSO (1 ml) was added at room temperature in an $N_2$ atmosphere a solution of $NaCH_2SOCH_3$ (2.0 M, 0.25 ml, 0.5 mmoles) in DMSO. After stirring for 10 minutes, MeI (38 μl, 0.6 mmoles) was added. After 20 minutes the reaction was diluted with $H_2O$ (15 ml) and extracted with $Et_2O$ (3 times). The combined ether extracts were

washed with $H_2O$ (3 times) and saturated brine and dried ($MgSO_4$). The ether was removed in vacuo to give 129 mg of a colorless syrup. A 50 mg sample was crystallized from hexane to give 25 mg of 1α-methoxy-2α,5β-bis-(3,4,5-trimethoxyphenyl)cyclopentane, m.p. 76-78.5°. NMR ($CDCl_3$): δ 6.56, 6.52 (s, 2H each, ArH), 3.82-3.92 (ms, 18H, $ArOCH_3$), 3.74 (m, 1H, $CHOCH_3$), 3.09 (s, 3H, $CHOCH_3$).

Using the same procedure the following ethers were prepared (non-crystalline compounds were purified by preparative TLC on silica gel with hexaneethyl acetate in varying ratios):

Scheme (f):

| R | X | m.p. | NMR ($CDCl_3$, δ (ppm)) |
|---|---|---|---|
| $CH_2C{\equiv}CH$ | Br | oil | 6.58, 6.56 (s, 2H each, ArH), 4.06 (m, 2H, $OCH_2$), 3.82-3.90 (ms, 18H, $ArOCH_3$), 3.74 (m, 1H, $CHOCH_2$), 2.30 (t, 1H, $C{\equiv}CH$). |
| $CH_2CO_2Et$ | Br | oil | 6.57, 6.61 (s, 2H each, ArH), 4.05 (q, 2H, $OCH_2CH_3$), 3.82-3.92 (ms, 19H, ArOCH3 + $CHOCH_2$), 3.62 (s, 2H, $OCH_2CO$), 1.15 (t, 3H, $OCH_2CH_3$). |
| $CH_2CH{=}CH_2$ | Br | oil | 6.58, 6.52 (s, 2H each, ArH), 5.65, 5.06, 4.97 (m, 1H each, $CH{=}CH_2$), 3.82-3.90 (ms, 18H, $ArOCH_3$), 3.70 (m, 1H, $CHOCH_2$), 3.62 (m, 2H, $CH_2CH{=}CH_2$). |
| $CH_2$ 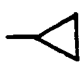 | Br | oil | 6.52, 6.58 (s, 2H each ArH), 3.82-3.90 (ms, 18H, $ArOCH_3$), 3.78 (m, 1H, $CHOCH_2$), 2.6-3.2 (12 peak multiplet (restricted rotation), 2H, $OCH_2$ 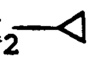 ), 0.88, 0.34, -.09 (m, 5H, 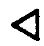 ). |

| R | X | m.p. | NMR ($CDCl_3$, δ (ppm)) |
|---|---|---|---|
| $CH_2CH_3$ | I | oil | 6.53, 6.59 (s, 2H each, ArH), 3.82-3.90 (ms, 18H, $ArOCH_3$), 3.78 (m, 1H, $CHOCH_2$), 2.9-3.35 (m, 4H, ArCH + $OCH_2CH_3$), 0.97 (t, 3H, $OCH_2CH_3$). |
| $CH_2CH_2CH_3$ | I | oil | 6.53, 6.57 (s, 2H each, ArH), 3.82-3.90 (ms, 18H, $ArOCH_3$), 3.76 (m, 1H, $CHOCH_2$), 2.86-3.30 (m, 4H, ArCH + $OCH_2CH_2$), 1.37 (m, 2H, $OCH_2CH_2CH_3$), 0.72 (t, 3H, $CH_2CH_3$). |
| $CH_2Ph$ | Br | oil | 7.18, 6.97 (m, 5H, Ph), 6.48, 6.57 (s, 2H each, ArH), 4.18 (q (restricted rotation), 2H, CH Ph), 3.76-3.92 (ms, 19H, $ArOCH_3$ + $CHOCH_2$). |
| $CH_2CH_2OCH_3$ | Cl | oil | 6.59, 6.57 (s, 2H each, ArH), 3.78-3.96 (ms, 19H, $ArOCH_3$ + $CHOCH_2$), 3.05-3.40 (m, 6H, ArCH + $OCH_2CH_2O$), 3.20 (s, 3H, $CH_2OCH_3$). |
| $CH_2\overset{O}{\overset{\Vert}{C}}N(CH_3)_2$ | Cl | 112-114° | 6.57, 6.56 (s, 2H each, ArH), 3.74-3.98 (ms, 21H, $ArOCH_3$ + $OCH_2CO$ + $CHOCH_2$), 2.77, 2.48 (s, 3H each, $N(CH_3)_2$). |

EXAMPLE 2

2-(3-Methoxy-5-(methylthio)-4-propoxyphenyl)-5-(3,4,5-trimethoxyphenyl)cyclopentanone and 2-(3-bromo-5-methoxy-4-propoxyphenyl)-5-(3,4,5-trimethoxyphenyl)-cyclopentanone

Step A: Preparation of 3-bromo-5-methoxy-4-propoxybenzaldehyde

50 g (0.43 mole) of 5-bromovanillin in 130 ml of DMF was added 150 g (1.08 mole) of potassium carbonate and 55 ml (0.606 mole) of bromopropane. The mixture was stirred for 72 hours at 70° C. The solution was then poured into 1 liter ice water. The aqueous solution was extracted with 3 × 200 ml $CH_2Cl_2$, the combined organic extracts were dried over $MgSO_4$. The solution was filtered and the filtrate was concentrated to dryness to yield 66.80 g of 3-bromo-5-methoxy-4-propoxybenzaldehyde as a yellow oil. ( 95% yield).

NMR ($CDCl_3$): δ 1.03 (t, 3H, term. $CH_3$), 1.83 (m, 2H, $OCH_2CH_2CH_3$), 3.90 (s, 3H, $OCH_3$), 4.08 (t, 2H, $OCH_2CH_2CH_3$), 7.34 ( d, 1H, 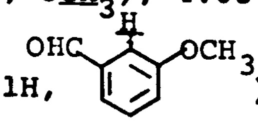 ), 7.60 (d, 1H, 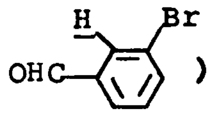 ), 9.83 (s, 1H, $-\overset{\overset{O}{\|}}{C}-H$).

Step B: Preparation of 3-methoxy-5-methylthio-4-propoxybenzyl alcohol

50 ml (0.56 mole) of methyl disulfide in 400 ml of 2.4-lutidine was added 46 g (0.72 mole) of copper. The mixture was heated at 140° for 3 hours. The solution was cooled to room temperature and 74.90 g (0.56 mole) of 3-bromo-5-methoxy-4-propoxybenz aldehyde was then added. The mixture was refluxed for 16 hours at 175° C. The solution was filtered and the filtrate was concentrated to dryness. The product was purified by flash column chromatography first then recrystallized from hexane to give 17.01 g (26% yield) of 3-methoxy-5-methylthio-4-propoxybenzaldehyde as a white solid. m.p. 64-66° C.

NMR ($CDCl_3$): δ 1.1 (t. 3H, term $CH_3$), 1.79 (m, 2H, $OCH_2CH_2CH_3$), 2.47 (s, 3H, $SCH_3$), 3.87 (s, 3H, $OCH_3$), 4.05 (t, 2H, $OCH_2CH_2CH_3$), 7.20 (S, 2H, Ar-H), 9.90

(S, 1H, $\overset{\overset{O}{\|}}{C}-H$).

15.5 g (0.065 mole) of 3-methoxy-5-methyl-thio-4-propoxybenzaldehyde in 300 ml of methanol was cooled in ice bath. 1 g (0.026 mole) of sodium borohydride was added in 3 portions to the above solution. The mixture was stirred for 10 minutes in ice bath. MeOH was removed, and the residue was purified by flash column chromatography to afford 15.45 g ( 95% yield) of 3-methoxy-5-methylthio-4-propoxybenzyl alcohol as a light yellow oil. NMR ($CDCl_3$): δ 1.06 (t, 3H, term $CH_3$), 1.90 (m, 2H, $-OCH_2CH_2CH_3$), 2.43 (s, 3H, $SCH_3$), 3.86 (s, 3H, $OCH_3$), 3.97 (t, 2H, $OCH_2CH_2CH_3$), 4.63 (d, 2H, $ArCH_2OH$), 6.74 (s, 2H, Ar-H).

Similarly, starting with 3-bromo-5-methoxy-4-propoxybenzaldehyde and following only the sodium borohydride procedure of Step B, 3-bromo-5-methoxy-4-propoxybenzyl alcohol was prepared as a yellow oil in a 95% yield.

NMR ($CDCl_3$): δ 1.05 (m, 3H $-OCH_2CH_2CH_3$), 4.55 (s, 2H, $Ar-CH_2-OH$), 6.80 (d, 1H, Ar-H), 7.05 (d, 1H, Ar-H).

Step C: Preparation of 3-methoxy-5-methylthio-4-propoxyphenylacetonitrile

15.45 g (0.064 mole) of 3-methoxy-5-methylthio-4-propoxybenzyl alcohol was cooled in ice bath and 4.9 ml (0.065 mole) of thionyl chloride was added. The mixture was stirred for 30 minutes in ice bath. Solvent and the excess thionyl chloride were removed under vacuum. Because the product 3-methoxy-5-methylthio-4-propoxybenzyl chloride was a heat sensitive compound, the residue (16 g) was used in the next step without further purification.

To 16 g of 3-methoxy-5-methylthio-4-propoxybenzyl chloride in 100 ml of dry DMF was added 5 g (0.10 mole) sodium cyanide. The mixture was stirred for about 48 hours at room temperature under $N_2$. The solution was filtered, and the filtrate was concentrated under vacuum. The residue was purified by flash column chromatography to yield 14.5 g of 3-methoxy-5-methylthio-4-propoxyphenyl acetonitrile as an oil. (95% yield).

NMR ($CDCl_3$): δ 3.65 (s, 2H, $CH_2CN$),, 1.04 (t, 3H, term $CH_3$), 1.80 (m, 2H, $OCH_2CH_2CH_3$), 2.40 (s, 3H, $SCH_3$), 3.85 (s, 3H, $OCH_3$), 3.95 (t, 2H, $OCH_2CH_2CH_3$), 6.66 (s, 2H, Ar-H).

Similarly, following the procedure of Step C, but substituting 3-bromo-5-methoxy-4-propoxyphenylbenzyl alcohol for 3-methoxy-5-(methylthio)-4-propoxybenzyl alcohol, afforded 3-bromo-5-methoxy-4-propoxyphenylacetonitrile as a yellow oil in a 95% yield.
NMR ($CDCl_3$): δ 1.06 (m, 3H, $-OCH_2CH_2CH_3$), 3.65 (s, 2H, $-CH_2$-CN), 6.78 (d, 1H, Ar-H), 7.05 (d, 1H, Ar-H).

Step D: Preparation of Ethyl 3-Methoxy-5-methylthio-4-propoxy-α-cyanobenzeneacetate (V)

1.4 g of sodium was dissolved in absolute EtoH with warming to 70 ° C, then EtOH was removed under vacuum to give a white solid residue of NaOEt. A mixture of 35 ml of diethyl carbonate, 43 ml of toluene, 13.46 g of 3-methoxy-5-methylthio-4-propoxyphenyl acetonitrile was added slowly. The mixture was refluxed for 3 hours. EtOH was removed under vacuum. The solid was filtered and washed with toluene (3×) and ether (3×). The solid was suspended in 200 ml of $H_2O$ and acidified with 6N HCl. The solid dissolved and a brown oil formed. The oil was extracted with ether (3×), the combined ether extracts were washed with $H_2O$ (2×), saturated NaCl (2×) and finally dried over $MgSO_4$. The ether was removed under vacuum to give 16 g (92% yield) of ethyl 3-methoxy-5-methylthio-4-propoxy-α-cyanobenzeneacetate.
NMR ($CDCl_3$): δ 4.75

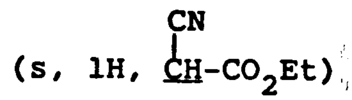

1.23 (m, 6H, $COOCH_2CH_3$, $OCH_2CH_2CH_3$), 1.80 (m, 2H, $OCH_2CH_2CH_3$), 2.43 (s, 3H, $SCH_3$), 3.83 (s, 3H, $OCH_3$), 4.2

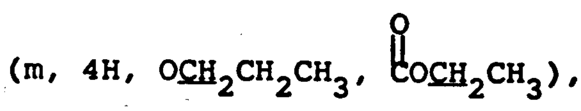

6.73 (s, 2H, Ar-H).

Similarly, following the procedure of Step D, but substituting 3-bromo-5-methoxy-4-propoxyphenylacetonitrile obtained in Step C for 3-methoxy-5-(methylthio)-4-propoxyphenylacetonitrileafforded ethyl 3-bromo-5-methoxy-4-propoxy-α-cyanobenzeneacetate as a yellow oil in a 60% yield.
NMR ($CDCl_3$): δ 1.05 (t, 3H, $-OCH_2CH_2CH_3$), 1.28 (t, 3H $-CO_2CH_2CH_3$), 3.88 (s, 3H, Ar-$OCH_3$), 3.95 (t, 2H, $-OCH_2CH_2CH_3$), 4.24 (m, 2H, $-CO_2CH_2CH_3$), 4.60 (s, 1H, -CH(CN)$CO_2$Et), 6.90 (d, 1H, Ar-H), 7.18 (d, 1H, Ar-H).

Following procedures similar to that of Step D but substituting 3,4,5-trimethoxyphenylacetonitrile for 3-methoxy-5-(methylthio)-4-propoxyphenylacetonitrile, there was prepared ethyl 3,4,5,-trimethoxy-α-cyanobenzeneacetate in 89% yield as a clear oil.
NMR ($CDCl_3$): δ 1.35 (t, 3H, $-CO_2CH_2CH_3$), 4.25 (q, 2H, $-CO_2CH_2CH_3$), 4.65 (s, 1H, -CH(CN)$CO_2$Et), 6.05 (s, 2H, Ar-H).

Step E: Preparation of Ethyl 3,4,5-trimethoxy-α-(2-bromoethyl)-α-cyanobenzeneacetate

To dry ethanol (100 ml) under a nitrogen atmosphere was added sodium (4.2 gm, 0.18 mol). After the sodium had completely dissolved, ethyl 3,4,5,-trimethoxy-α-cyanobenzeneacetate (50.0 g, 0.18 mol) in ethanol (70 ml) was added dropwise over 30 minutes. A slight exotherm was observed. The reaction was heated to 90 ° C for 30 minutes, then added dropwise over 2 hours to a refluxing solution of dibromoethane (340.0 g, 1.8 mol) in ethanol (500 ml). The reaction was refluxed an additional 3 hours, then concentrated. The residue was taken up in water (300 ml) and extracted with ethyl acetate (2 × 500 ml). The combined organic layers were washed with brine (1 × 100 ml), dried ($MgSO_4$) and concentrated to a solid. The solid was washed with hexane (2 × 200 ml) then recrystallized from hexane to give ethyl 3,4,5-trimethoxy-α-(2-bromoethyl)-α-cyanobenzeneacetate in 65% yield as a white solid, m.p. 76-77 ° C.
NMR ($CDCl_3$): δ 1.30 (t, 3H, $-CO_2CH_2CH_3$), 4.25 (q, 2H, $CO_2CH_2CH_3$), 4.62 (s, 1H, -CH(CN)$CO_2$Et), 6.65 (s, 2H, Ar-H).

Following procedures similar to the procedure of Step E but substituting ethyl 3-bromo-5-methoxy-4-propoxy-α-cyanobenzeneacetate for ethyl 3,4,5-trimethoxy-α-cyanobenzene acetate, there was prepared ethyl 3-bromo-5-methoxy-4-propoxy-α-(α-bromoethyl)-α-cyanobenzeneacetate in a 92% yield as a light yellow oil.
NMR ($CDCl_3$): δ 1.04 (t, 3H, $-OCH_2CH_2CH_3$), 1.28 (t, 3H, $-CO_2CH_2\underline{CH_3}$), 3.88 (s, 3H, Ar-$OCH_3$), 3.96 (t, 2H, $O\underline{CH_2}CH_2CH_3$), 6.48 (d, 1H, Ar-H), 7.28 (d, 1H, Ar-H).

<u>Step F</u>: <u>Preparation of Diethyl 2-(3-Methoxy-5-(methylthio)-4-propoxyphenyl)-2,5-dicyano-5-(3,4,5-trimethoxyphenyl)-1,6-hexanedioate</u>

To a slurry of sodium hydride (0.65 g, 0.027 mol) in dimethylformamide (50 mL) was added dropwise ethyl 3-methoxy-5-(methylthio)-4-propoxy-α-cyanobenzeneacetate (6.8 g, 0.021 mol) in dimethylformamide (15 ml) over 40 minutes at room temperature under nitrogen. The reaction was heated to 90°C for 10 minutes, then ethyl 3,4,5-trimethoxy-2-(2-bromoethyl)-α-cyanobenzeneacetate (8.16 g, 0.021 mol) in dimethylformamide (20 ml) was slowly added. The reaction was kept at 95°C for four hours then cooled, filtered and concentrated. The crude oil was purified by flash column (30% ethyl acetate/hexane as eluant) to afford diethyl 2-(3-methoxy-5-(methylthio)-4-propoxyphenyl)-2,5-dicyano-5-(3,4,5-trimethoxyphenyl)-1,6-hexanedioate in 62% yield as a white solid (m.p. 131-132.5°C).

NMR ($CDCl_3$): δ 1.04 (t, 3H, term $C\underline{H}_3$), 1.26 (m, 6H $-\overset{O}{\overset{\|}{C}}OCH_2\underline{C}\underline{H}_3$), 1.82 (m, 2H, $OCH_2C\underline{H}_2CH_3$), 2.20 (2H, m, $Ar\text{-}CH_2\text{-}Ar$), 2.44 (m+s, $S C\underline{H}_3$ + $Ar\text{-}CH_2\text{-}Ar$ ), 3.86 (m, 12H, $OC\underline{H}_3$), 3.96 (m, 2H, $O\underline{CH}_2CH_2CH_3$), 4.26 (q, 4H, $-\overset{O}{\overset{\|}{C}}-OC\underline{H}_2CH_3$), 6.66, 6.74 (s, 2H, 2Ar-H), 6.75, 6.79 (d,d, 2H, ArH).

Following substantially the procedure of Step F but substituting ethyl 3,4,5-trimethoxy-α-cyanobenzeneacetate for ethyl 3-methoxy-5-(methylthio)-4-propoxy-α-cyanobenzeneacetate and substituting ethyl 3-bromo-5-methoxy-4-propoxy-α-(2-bromoethyl)-2-cyanobenzeneacetate for ethyl 3,4,5-trimethoxy-2-(2-bromoethyl)-α-cyano-benzenacetate, there was prepared diethyl 2-(3-bromo-5-methoxy-4-propoxyphenyl)-2,5-dicyano-5-(3,4,5-trimethoxyphenyl)-1,6-hexanedioate as an oil in a 63% yield. The product is a mixture of diastereoisomers.
NMR ($CDCl_3$): δ 1.25 (m, 3H, $-OCH_2CH_2CH_3$), 1.26 (m, 3H, $-CO_2CH_2CH_3$), 6.68 (s, Ar-H), 6.74 (s, Ar-H), 6.92 (d, Ar-H), 6.97 (d, Ar-H), 7.22 (d, Ar-H), 7.31 (d, Ar-H).

<u>Step G</u>: <u>Preparation of 2-(3-methoxy-5-(methylthio)-4-propoxyphenyl)-5-(3,4,5-trimethoxyphenyl)-1,6-hexanedinitrile</u>

To a solution of diethyl 2-(3-methoxy-5-(methylthio)-4-propoxyphenyl)-2,5-dicyano-5-(3,4,5-trimethoxyphenyl)-1,6-hexanedioate (12.0 g, 0.019 mol) in ethanol (250 ml) was added potassium hydroxide (4.4 g, 0.079 mol). The reaction was heated to 95°C for 20 minutes, then filtered, and the filtrate concentrated. Methanol (300 ml) was added to the residue, and the solution was stirred at room temperature overnight. The reaction was filtered and the precipitate dried to yield 2-(3-methoxy-5-(methylthio)-4-propoxyphenyl)-5-(3,4,5-trimethoxyphenyl)-1,6-hexanedinitrile in a 66% yield. (m.p. 131-132.5°C).

NMR ($CDCl_3$): δ 1.04 (t, 3H, term $CH_3$), 1.80 (m, 2H, $OCH_2CH_2CH_3$), 2.18 (m, 2H, $CH_2$ from 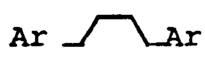 ), 2.44 (m+s, $SCH_3$ + $CH_2$ from 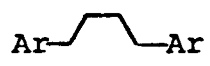 ), 3.90 (m, 14H, $-OCH_3$ + $OCH_2CH_2CH_3$), 6.64 + 6.72 (s, 2H, ArH), 6.71, 6.77, 6.81, 6.83 (d,d, 2H, ArH).

Following substantially the same procedure of Step G, but substituting diethyl 2-(3-bromo-5-methoxy-4-propoxyphenyl)-2,5-dicyano-5-(3,4,5-trimethoxyphenyl)-1,6-hexanedioate for diethyl 2-(3-methoxy-5-(methylthio)-4-propoxyphenyl)-2,5-dicyano-5-(3,4,5-trimethoxyphenyl)-1,6-hexanedioate there was obtained 2-(3-bromo-5-methoxy-4-propoxyphenyl)-(3,4,5-trimethoxyphenyl)-1,6-hexanedinitrile as an oil in 30% yield.
NMR ($CDCl_3$): δ 1.05 (t, 3H, $-OCH_2CH_2CH_3$), 1.82 (m, 2H $-OCH_2CH_2CH_3$), 3.95 (t, 2H, $-OCH_2CH_2CH_3$), 6.50 (s, 2H, Ar-H), 6.95 (d, 1H, Ar-H), 7.05 (d, 1H, Ar-H).

Step H: Preparation of 2-(3-methoxy-5-methylthio-4-propoxyphenyl)-5-(3,4,5-trimethoxyphenyl)-1,6-hexanedioic acid

A solution of 2-(3-methoxy-5-(methylthio)-4-propoxyphenyl)-(3,4,5-trimethoxyphenyl)-1,6-hexanedinitrile (3.0 g, 0.0062 mol) in hydrochloric acid saturated methanol (240 ml) was stirred overnight at room temperature. The reaction was concentrated, and the residue taken up in water (200 ml) and stirred at room temperature for 40 hours. The resulting precipitate was filtered and washed with water then dissolved in water/ethanol 4:1 (150 ml) containing 3.4 g of potassium hydroxide. The solution was heated to 95 °C for 18 hours, cooled, filtered, and the filtrate neutralized with 37% hydrochloric acid. The resulting precipitate was washed with water and dried to yield 2-(3-methoxy-5-(methylthio)-4-propoxyphenyl)-5-(3,4,5-trimethoxyphenyl)-1,6-hexanedioic acid as a white solid (67%). m.p. = 172-173.5 °C.

NMR ($CDCl_3$): δ 1.03 (t, 3H, term $CH_3$), 1.8-2.0 (m, 6H, $OCH_2CH_2CH_3$, 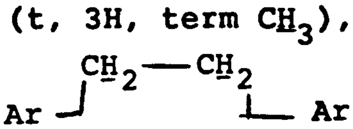 ), 2.37 (s, 3H, $SCH_3$), 3.50 (m, 2H, ArCH-), 3.80 (m, 12H, $OCH_3$), 3.90 (2H, t, $OCH_2CH_2CH_3$), 6.48 (s, 2H, ArH), 6.61 (s, 2H, ArH).

Following substantially the same procedure of Step H but substituting 2-(3-bromo-5-methoxy-4-propoxyphenyl)-(3,4,5-trimethoxyphenyl)-1,6-hexanedinitrile for 2-(3-methoxy-5-(methylthio)-4-propoxyphenyl)-(3,4,5-trimethoxyphenyl)-1,6-hexanedinitrile there was obtained 2-(3-bromo-5-methoxy-6-propoxyphenyl)-5-(3,4,5-trimethoxyphenyl)-1,6-hexanedioic acid in a 69% yield as an oil.
NMR ($CDCl_3$): δ 1.03 (t, 3H, $-OCH_2CH_2CH_3$), 3.36-3.55 (m, 2H, $(-CH(CO_2H)-)$, 6.48 (m, 2H, Ar-H), 6.72 (m, 1H, Ar-H), 7.05 (m, 1H, Ar-H).

Step I: Preparation of 2-(3-methoxy-5-(methylthio)-4-propoxyphenyl)-5-(3,4,5-trimethoxyphenyl)-cyclopentanone

A solution of 2-(3-methoxy-5-(methylthio)-4-propoxyphenyl)-5-(3,4,5-trimethoxyphenyl)-1,6-hexanedioic acid (0.86 g, 1.6 mmol) and acetic anhydride (5 ml) was heated to 130-135 °C for 30 minutes, then the excess solvent was removed. The pale yellow oil residue was then heated for 6 minutes at 275-280 °C, cooled, and purified by preparative TLC developed with 30% ethyl acetate-hexane to afford a 44% yield of

2-(3-methoxy-5-(methylthiol)-4-propoxyphenyl)-5-(3,4,5-trimethoxyphenyl)-cyclopentanone as a 1:4 cis:trans mixture.
Mass spectrum; m/e 460 ($M^+$)
NMR ($CDCl_3$): δ 1.40 (t, 3H, term, $CH_3$, 1.80 (m, 2H, $OCH_2CH_2CH_3$), 2.15 (m, 2H, cyclopentane $CH_2$), 2.41 (s, 3H, $SCH_3$), 2.61 (m, 2H, cyclopentane $CH_2$), 3.40-3.60 (broad m, 2H, ArCH), 3.76-3.96 (m, 14H, 4-$OCH_3$ + $OCH_2CH_2CH_3$), 6.39, 6.48, 6.54, 6.64 (s, 4H, Ar-H).

Following substantially the same procedure of Step I but substituting 2-(3-bromo-5-methoxy-6-propoxyphenyl)-5-(3,4,5-trimethoxyphenyl)-1,6-hexanedioic acid for 2-(3-methoxy-5-(methylthio)-4-propoxyphenyl)-5-(3,4,5-trimethoxyphenyl)-1,6-hexanedioic acid there was prepared a 1:4 cis:trans mixture of 2-(3-bromo-5-methoxy-4-propoxyphenyl-5-(3,4,5-trimethoxyphenyl)cyclopentanone as an oil in a 15% yield.
NMR ($CDCl_3$): δ trans isomer δ 1.06 (m, 3H, $-OCH_2CH_2CH_3$), 3.55-3.50 (m, 2H, cyclopentyl α-H), 6.47 (s, 2H, Ar-H), 6.80 (d, 1H, Ar-H), 7.00 (d, 1H, Ar-H).
cis isomer δ 1.06 (m, 3H, $-OCH_2CH_2CH_3$), 3.50-3.60 (m, 2H, cyclopentyl α-H), 6.32 (s, 2H, Ar-H), 6.70 (d, 1H, Ar-H), 7.00 (d, 1H, Ar-H).

## EXAMPLE 3

### 2-(3-Methoxy-5-(methylthio)-4-propoxyphenyl)-5-(3,4,5-trimethoxyphenyl)cyclopentanol

To a solution of 2-(3-methoxy-5-(methylthio)-4-propoxyphenyl)-5-(3,4,5-trimethoxyphenyl)-cyclopentanone (0.40 g, 0.87 mmol) in methanol (20 ml) was added sodium borohydride (0.10 mg, 2.6 mmol). The reaction was refluxed for 30 minutes, then quenched into water (50 ml) and extracted with ether (3 × 50 ml). The combined organic extracts were washed with brine (50 ml), dried ($MgSO_4$) and concentrated to give a total yield of 55% of 2-(3-methoxy-5-)methylthiol)-4-propoxyphenyl)-5-(3,4,5-trimethoxyphenyl)cyclopentanol as a 1:2:2 mixture of the isomers A, B, and C. The isomers were separated by preparative TLC developed with 30% ethyl acetate-hexane.

Physical data of the Isomers:

A. 2α-(3-methoxy-5-methylthio-4-propoxyphenyl)-5α-(3,4,5-trimethoxyphenyl)-1α-cyclopentanol.
m.p. = 94-95.5°C
mass spectrum: m/e 462 ($M^+$)
B. 2β-(3-methoxy-5-methylthio-4-propoxyphenyl-5α-(3,4,5-trimethoxyphenyl)-1α-cyclopentanol.
m.p. = 102.5-104.5°C
mass spectrum: m/e 462 ($M^+$)
Analysis = $C_{25}H_{34}O_6S \bullet 1/4\ H_2O$ (M.W. = 467.1)
Calc for: C, 64.28; H, 7.44.
Found: C, 64.26; H, 7.43.
C. 2α-(3-methoxy-5-methylthio-4-propoxyphenyl)-5β-(3,4,5-trimethoxyphenyl)-1α-cyclopentanol.
m.p. = 98-99°C
mass spectrum: m/e 462 ($M^+$)
Analysis = $C_{25}H_{34}O_6S$ (M.W. = 462.60)
Calc for: C, 64.91; H, 7.41.
Found: C, 65.31; H, 7.42.

## EXAMPLE 4

### 2-(3-Bromo-5-methoxy-4-propoxyphenyl)-5-(3,4,5-trimethoxyphenyl)cyclopentanol

Following substantially the same procedure of Example 3, but substituting 2-(3-bromo-5-methoxy-4-propoxyphenyl)-5-(3,4,5-trimethoxyphenyl)-cyclopentanone for 2-(3-methoxy-5-(methylthio)-4-propoxyphenyl)-5-(3,4,5-trimethoxyphenyl)cyclopentanone there was prepared 2-(3-bromo-5-methoxy-4-propoxyphenyl)-5-(3,4,5-trimethoxyphenyl cyclopentanol in a 50% yield of a 1:2:2 mixture of isomers (1α, 2α, 5α):-(1α, 2β, 5α):(1β, 2β, 5α).
The isomers were separated by preparative TLC developed with 30% ethyl acetate-hexane.
NMR ($CDCl_3$):
isomer (1α, 2α, 5α): δ 1.06 (t, 3H, $-OCH_2CH_2CH_3$), 1.84 (m, 2H, $-OCH_2CH_2CH_3$), 3.20-3.40 (m, 2H, ArCH, 4.25-4.30 (m, 1H, cyclopentyl CHOH), 6.60 (s, 2H, Ar-H), 6.88 (d, 1H, Ar-H), 7.11 (d, 1H, Ar-H).

isomer (1α, 2β, 5α): δ 1.06 (t, 3H, $-OCH_2CH_2CH_3$), 1.84 (m, 2H, $-OCH_2CH_2CH_3$), 3.10-3.34 (m, 2H, ArCH, 4.12-4.14 (m, 1H, cyclopentyl CHOH), 6.52 (s, 2H, Ar-H), 6.74 (d, 1H, Ar-H), 7.08 (d, 1H, Ar-H).
isomer (1α, 2α, 5β): δ 1.06 (t, 3H, $-OCH_2CH_2CH_3$), 1.84 (m, 2H, $-OCH_2CH_2CH_3$), 3.08-3.34 (m, 2H, ArCH, 4.12-4.22 (m, 1H, cyclopentyl CHOH), 6.52 (s, 2H, Ar-H), 6.08 (d, 1H, Ar-H), 7.06 (d, 1H, Ar-H).

## EXAMPLE 5

1α-Methoxy-2α-(3-methoxy-5-methylthio-4-propoxyphenyl)-5β(3,4,5-trimethoxyphenyl)cyclopentane

Approximately 10 mg of sodium hydride in 1.5 ml of DMSO was heated at 75 °C for 40 minutes until the evolution of hydrogen ceased and the solution turned clear. To the above cooled sodium methylsulfinyl carbanion solution 30 mg of 2α-(3-methoxy-5-methylthio-4-propoxyphenyl)-5β-(2,3,4-trimethoxyphenyl)-1α cyclopentanol in 1 ml of DMSO was added slowly. The mixture was stirred for 20 minutes at room temperature until the color turned dark blue. Excess methyl iodide (500 µl) was added to the dark blue solution. The blue color disappeared immediately. The mixture was stirred for another 10 minutes at room temperature. The DMSO was removed and the residue was purified by preparation TLC (30% ethyl acetate-hexane) to yield 24 mg of 1α-methoxy-2α-(3-methoxy-5-methylthio-4-propoxyphenyl)-5β(3,4,5-trimethoxyphenyl)-cyclopentane. (73% yield)
Mass spectrum: m/e 476 ($M^+$)
NMR ($CDCl_3$): δ 3.09 (s, 3H, 1α-$OCH_3$)

Following essentially the same procedures as described above, there were prepared the following compounds:

(a) 1α-Methoxy-2α-(3-methoxy-5-methylthio-4-propoxyphenyl)-5α-(3,4,5-trimethoxyphenyl)cyclopentane (yield, 70%).
Mass spectrum: m/e 476 ($M^+$)
NMR ($CDCl_3$): δ 2.81 (s, 3H, 1α-$OCH_3$).
(b) 1α-Methoxy-2β-(3-methoxy-5-methylthio-4-propoxyphenyl)-5α-(3,4,5-trimethoxyphenyl)cyclopentane (yield, 79%);
Mass spectrum: m/e 476 ($M^+$)
NMR ($CDCl_3$): δ 3.08 (s, 3H, 1α-$OCH_3$).
(c) 1α-Methoxy-2β-(3-bromo-5-methoxy-4-propoxyphenyl) -5α-(3,4,5-trimethoxyphenyl)cyclopentane (yield, 56%);
NMR ($CDCl_3$): δ 3.08 (s, 3H, 1α-$OCH_3$).
(d) 1α-Methoxy-2β-(3-bromo-5-methoxy-4-propoxyphenyl)-5β-(3,4,5-trimethoxyphenyl)cyclopentane (yield, 65%);
NMR ($CDCl_3$): δ 3.07 (s, 3H, 1α,-$OCH_3$).

## EXAMPLE 6

1α-Methoxy-2β-(3-methoxy-5-methylsulfonyl-4-propoxyphenyl)-5α(3,4,5-trimethoxyphenyl)cyclopentane and the corresponding sulfinyl derivative

To a solution of 17 mg of 1α-methoxy-2β-(3-methoxy-5-methylthio-4-propoxyphenyl)-5α-(3,4,5-trimethoxyphenyl)cyclopentane in 2 ml of methylene chloride was added 80 mg of metachloroperoxybenzoic acid. The mixture was stirred at room temperature for about 30 minutes. The crude products were isolated and purified by preparative TLC (30% ethyl acetate-hexane) to yield the title compound in 58% yield;
Mass spectrum: m/e 509 ($M^+$); NMR ($CDCL_3$): δ 3.08 (s, 3H, 1α-$OCH_3$), 3.25 (s, 3H, $SO_2CH_3$) and the corresponding sulfinyl derivative in 19% yield, Mass spectrum: m/e 492 ($M^+$), NMR ($CDCl_3$): δ 3.08 (s, 3H, 1α-$OCH_3$), 2.79 (d, 3H, $SOCH_3$).

Following essentially the same procedures as described above, the following compounds were prepared:

(a) 1α-Methoxy-2α-(3-methoxy-5-methylsulfonyl-4-propoxyphenyl)-5α-(3,4,5-trimethoxyphenyl)-cyclopentane in 40% yield; Mass spectrum: m/e 509 ($M^+$);
NMR ($CDCl_3$): δ 2.76 (s, 3H, 1α-$OCH_3$), 3.24 (s, 3H, $SO_2CH_3$); m.p. 140.5-142 °C.
(b) 1α-Methoxy-2α-(3-methoxy-5-methylsulfonyl-4-propoxyphenyl)-5α-(3,4,5-trimethoxyphenyl)-cyclopentane in 30% yield; Mass spectrum: m/e 492 ($M^+$);
NMR ($CDCl_3$): δ 2.78 (d 3H, $SOCH_3$), 2.80 (s, 3H, 1α-$OCH_3$).

(c) 1α-Methoxy-2α-(3-methoxy-5-methylsulfonyl-4-propoxyphenyl)-5β-(3,4,5-trimethoxyphenyl)-cyclopentane in 30% yield; Mass spectrum: m/e 509 ($M^+$);
NMR ($CDCl_3$): δ 3.05 (s, 3H, 1α-$OCH_3$), 3.28 (s, 3H, $SO_2CH_3$).
(d) 1α-Methoxy-2α-(3-methoxy-5-methylsulfinyl-4-propoxyphenyl)-5β-(3,4,5-trimethoxyphenyl)-cyclopentane in 40% yield; Mass spectrum: m/e 492 ($M^+$);
NMR ($CDCl_3$): δ 3.07 (d, 3H, 1α-$OCH_3$), 2.83 (d, 3H, $SOCH_3$).

EXAMPLE 7

1α-Methoxy-2α-(5-cyano-3-methoxy-4-propoxyphenyl)-5β-(3,4,5-trimethoxyphenyl)cyclopentane

To a solution of 1α-Methoxy-2α-(5-bromo-3-methoxy-4-propoxyphenyl)-5β-(3,4,5-trimethoxyphenyl)-cyclopentane (22.0 mg, 0.043 mmol) in N-methylpyrrolidone (1.5 mL) was added cuprous cyanide (35.0 mg, 0.39 mmol). The reaction was heated to 175°C for 8 hours, cooled, quenched into water (20 ml) and filtered. The filtrate was extracted with methylene chloride (3 × 25 ml). The combined organic layers were washed with brine (1 × 20 ml) dried ($MgSO_4$) and concentrated. The resulting brown oil was purified by preparative TLC developed with 30% ethyl acetate-hexane. 1α-Methoxy-2α-(5-cyano-3-methoxy-4-propoxyphenyl)-5β-(3,4,5-trimethoxyphenyl)cyclopentane was obtained in a 51% yield as a clear oil.
NMR ($CDCl_3$): δ 1.06 (t, 3H, -$OCH_2CH_2CH_3$), 1.84 (m, 2H, -$OCH_2CH_2CH_3$), 3.08 (s, 3H, 1α-$OCH_3$), 3.68-3.78 (m, 1H, cyclopentyl $CHOCH_3$), 6.56 (s, 2H, Ar-H), 7.02-7.10 (m, 2H, Ar-H).

Following substantially the same procedures as described above there was prepared 1α-methoxy-2β-(5-cyano-3-methoxy-4-propoxyphenyl)-5α-(3,4,5-trimethoxyphenyl)cyclopentane in a 59% yield as a clear oil.
NMR ($CDCl_3$): δ 1.06 (t, 3H, -$OCH_2CH_2CH_3$), 1.84 (m, 2H, -$OCH_2CH_2CH_3$), 3.10 (s, 3H, 1α-$OCH_3$), 3.70-3.78 (m, 1H, cyclopentyl $CHOCH_3$), 6.30 (s, 2H, Ar-H), 7.04-7.12 (m, 2H, Ar-H).

EXAMPLE 8

1α-Methoxy-2α-(5-amido-3-methoxy-4-propoxyphenyl)-5β-(3,4,5-trimethoxyphenyl)cyclopentane

A solution of 1α-methoxy-2α-(5-cyano-3-methoxy-4-propoxyphenyl)-5β-(3,4,5-trimethoxyphenyl)-cyclopentane (7.0 mg, 0.015 mmol) in 10% sodium hydroxide in 3:1, ethanol:water (1.0 ml) was refluxed for 3-1/2 hours. The reaction was cooled, then quenched into water (5 ml) and extracted with methylene chloride (3 × 5 ml). The combined organic layers were washed with brine (1 × 5 ml) dried ($MgSO_4$) and concentrated to a clear oil. The oil was purified by preparative TLC developed with 55% ethyl acetate-hexane to afford a 56% yield of 1α-methoxy-2α-(5-amido-3-methoxy-4-propoxyphenyl)-5β-(3,4,5-trimethoxyphenyl)cyclopentane.
NMR ($CDCl_3$): δ 1.04 (t, 3H, -$OCH_2CH_2CH_3$), 3.08 (s, 3H, 1α-$OCH_3$), 4.04 (t, 2H, -$OCH_2CH_2CH_3$), 5.72 (m, 1H, -$CONH_2$), 6.57 (s, 2H, Ar-H), 7.00 (d, 1H, Ar-H), 7.65 (d, 1H, Ar-H), 7.99 (m, 1H, $CONH_2$).

Following essentially the same procedure as described above there was prepared the following compounds: 1α-methoxy-2β-(5-amido-3-methoxy-4-propoxyphenyl)-5α-(3,4,5-trimethoxyphenyl)cyclopentane in a 41% yield as a clear oil.
NMR ($CDCl_3$): δ 1.06 (t, 3, -$OCH_2CH_2CH_3$), 3.08 (s, 3H, 1α-$OCH_3$), 4.04 (t, 2H, -$OCH_2CH_2CH_3$) 5.79 (m, 1H, $CONH_2$), 6.52 (s, 2H, Ar-H), 7.10 (d, 1H, Ar-H), 7.65 (d, 1H, Ar-H), 8.02 (m, 1H, $CONH_2$).

EXAMPLE 9

**Trans-2,5-bis(3,4,5-trimethoxyphenyl)cyclopentanone-O-methyloxime**

A mixture of trans-2,5,-bis(3,4,5-trimethoxyphenyl)cyclopentanone (208 mg, 0.5 mmoles), NaOAc (82 mg, 1 mmole), $CH_3ONH_2$• HCl (83.5 mg, 0.5 mmole) and EtOH (1 ml) was heated at 90° in $N_2$ atmosphere with stirring for 4 hours. After standing overnight at room temperature the mixture was diluted with $H_2O$ (10 ml) and extracted with $CH_2Cl_2$ (threetimes). The combined $CH_2Cl_2$ extracts were washed with $H_2O$ and dried ($MgSO_4$). Evaporation of the $CH_2Cl_2$ in vacuo gave 223 mg of a colorless gum. Purification by preparative TLC (silica gel, hexane EtOAc, 1:1) gave 139 mg of trans-2,5,-bis-(3,4,5-trimethoxyphenyl)-cyclopentanone-O-methyloxime, m.p. 61-63°.

Using the same procedure trans-2,5-bis-(3,4,5-trimethoxyphenyl)cyclopentanone oxime was prepared.

EXAMPLE 10

**Trans-2,5-bis(3,4,5-trimethoxyphenyl)cyclopentanamine hydrochloride**

To a solution of trans-2,5-bis(3,4,5-trimethoxyphenyl)cyclopentanone-O-methyloxime (53 mg, 0.12 mmoles) in THF (1 ml) cooled in an ice bath was added $BH_3$•THF (1M, 0.5 ml, 0.5 mmoles). The solution was heated in a $N_2$ atmosphere at 55° for 20 hours. The clear solution was cooled in an ice bath and 3 ml of 10% NaOH was added. After dilution with $H_2O$ (10 ml), the mixture was extracted with $Et_2O$ (three times). The combined $Et_2O$ extracts were shaken with 0.5M HCl (three times). The combined acidic extracts were basified with 50% NaOH and extracted with $Et_2O$ (three times). The combined $Et_2O$ extracts were washed with $H_2O$ and saturated brine and dried ($K_2CO_3$). Evaporation of the solvent in vacuo gave 19 mg of a colorless oil. This material was dissolved 10 ml of $Et_2O$ and a stream of HCl gas was bubbled in a few seconds. The $Et_2O$ was evaporated in vacuo to give 20 mg of trans-2,5-bis(3,4,5-trimethoxyphenyl)-cyclopentanamine hydrochloride, m.p. 270-272° dec.

EXAMPLE 11

1a,3β-bis(3,4,5-trimethoxyphenyl)cyclopentane

Step A: Preparation of trans-2,5-bis(3,4,5-trimethoxyphenyl)cyclopentanone-p-toluenesulfonylhydrazone

A mixture of trans-2,5-bis(3,4,5-trimethoxyphenyl)cyclopentanone (97 mg, 0.23 mmoles), p-toluenesulfonylhydrazine (52 mg, 0.27 mmoles) and EtOH (1.0 ml) was heated under reflux in a $N_2$ atmosphere for 5 hours. The cooled reaction mixture was filtered, and the solid washed with cold EtOH (two times) and dried to give 85 mg of trans-2,5-bis(3,4,5-trimethoxyphenyl)cyclopentanone p-toluenesulfonylhydrazone.

Step B: Preparation of 1α,3β-bis(3,4,5-trimethoxyphenyl)cyclopentane

A mixture of trans-2,5-bis(3,4,5-trimethoxyphenyl)cyclopentanone p-toluenesulfonylhydrazone (80 mg, 0.137 mmoles), $NaBH_3CN$ (35 mg, 0.548 mmoles), p-toluenesulfonic acid (7 mg), DMF (0.34 ml) and sulfolane (0.34 ml) was heated at 110° in an $N_2$ atmosphere with stirring for 2 hours. Over the next two days the same amounts of $NaBH_3CN$ and p-toluenesulfonic acid were added followed by heating for 2-3 hours a total of five more times. The reaction mixture was diluted with $H_2O$ (15 ml) and extracted with $CH_2Cl_2$ (four times). The $CH_2Cl_2$ extracts were washed with $H_2O$ (three times) and dried ($MgSO_4$). Evaporation of the solvent in vacuo gave 62 mg of a brown oil. Purification by preparative TLC (silica gel, hexane-EtOAc, 1:1) gave 13 mg of 1α,3β-bis(3,4,5-trimethoxyphenyl)cyclopentane as a colorless oil; mass spectrum: m/e 402 ($M^+$).

EXAMPLE 12

1α-acetoxy-2α,5β-bis(3,4,5-trimethoxyphenyl)cyclopentane

To a solution of trans-2,5-bis(3,4,5-trimethoxyphenyl)cyclopentanol (41.8 mg, 0.1 mmole), 4-dimethylaminopyridine (12 mg, 0.1 mmole) in $CH_2Cl_2$ (0.2 ml) was added acetic anhydride (19 μl, 0.2 mmole). The mixture was allowed to stand at room temperature for 20 hours. It was diluted with EtOAc and purified by preparative TLC (silica gel, hexane-EtOAc, 1:1) to give 1a-acetoxy-2α,5β-bis(3,4,5-trimethoxyphenyl)cyclopentane as a colorless syrup. NMR ($CDCl_3$): δ 6.58, 6.51 (s, 2H each, ArH), 5.36 (m, 1H, CHOAc), 3.80-3.90 (ms, 18H, $ArOCH_3$), 2.20

$$(s, 3H, O\overset{O}{\overset{\|}{C}}CH_3).$$

EXAMPLE 13

1α-(2α,5β-bis(3,4,5-Trimethoxyphenyl)cyclopentyl)oxy)acetic acid

A mixture of ethyl 1α-((2α,5β-bis-(3,4,5-trimethoxyphenyl)cyclopentyl)oxy)acetate (20 mg); MeOH (5 ml),

and 50% NaOH (0.1 ml) was stirred at room temperature in a $N_2$ atmosphere for 4 hours. Most of the MeOH was removed on a rotary evaporator (bath 50°). The clear solution was diluted with $H_2O$ (10 ml), extracted with $Et_2O$, and acidified with concentrated HCl. The cloudy solution was extracted with $Et_2O$ (3 times). The combined $Et_2O$ extracts were washed with $H_2O$ and saturated brine and dried ($MgSO_4$). Removal of the solvent in vacuo gave 13 mg of 1α-((2α,5β-bis(3,4,5-trimethoxyphenyl)cyclopentyl)oxy)acetic acid as a colorless syrup. NMR ($CDCl_3$): δ 6.53, 6.48 (s, 2H each, ArH), 3.82-3.90 (ms, 19H, $ArOCH_3$ + $CHOCH_2$), 3.78 (q, restricted rotation, 2H, $OCH_2CO$).

EXAMPLE 14

1α-N,N-dimethylaminoethoxy-2α,5β-bis(trimethoxyphenyl)cyclopentane

To a solution of 1α-((2α,5β-bis(3,4,5-trimethoxyphenyl)cyclopentyl)oxy)-N,N-dimethylacetamide (135 mg, 0.27 mmoles) in THF (4 ml) cooled in an ice bath was added $LiAlH_4$ (35 mg, 0.92 mmole). The suspension was stirred at room temperature in a $N_2$ atmosphere for 3 hours. It was then heated at 55° for 1 hour. After cooling in an ice bath, $H_2O$ (35 µl) was added cautiously followed by 15% NaOH (35 µl) and $H_2O$ (105 µl). The suspension was stirred at room temperature for 30 minutes. The solid was filtered and washed with THF. The filtrate and washes were dried ($K_2CO_3$) and evaporated in vacuo to give 121 mg of a colorless oil. This material was dissolved in $Et_2O$ (15 ml) and extracted with 1N HCl (three times). The combined acidic extracts were washed with $Et_2O$ and basified with 50% NaOH> The cloudy solution was extracted with $CH_2Cl_2$ (three times), and the combined extracts were washed with water and saturated brine and dried ($K_2CO_3$). Evaporation in vacuo gave 112 mg of a colorless oil. This material was purified by preparative TLC (silica gel; $CHCl_3$, acetone, $Et_2NH$; 10:4:1) to give 78 mg of 1α-N,N-dimethylaminoethoxy-2α,5β-bis(trimethoxyphenyl)cyclopentane as a colorless syrup. NMR ($CDCl_3$): δ 6.58, 6.52 (s, 2H each, ArH), 3.74-3.90 (ms, 19H, $ArOCH_3$ + $CHOCH_2$), 3.00-350 (m, 6H, ArCH + $OCH_2CH_2N$), 2.11 (s, 6H, $N(CH_3)_2$).

C. Utility of the compounds within the scope of the invention

This invention also relates to a method of treatment for patients (or mammalian animals raised in the dairy, meat, or fur industries or as pets) suffering from disorders or diseases which can be attributed to PAF as previously described, and more specifically, a method of treatment involving the administration of the PAF antagonists of formula (I) as the active constituents.

Accordingly, the compounds of Formula (I) can be used among other things to reduce pain and inflammation, to correct respiratory, cardiovascular, and intravascular alterations or disorders, and to regulate the activation or coagulation of platelets, to correct hypotension during shock, the pathogenesis of immune complex deposition and smooth muscle contractions.

For the treatment of inflammation such as rhumatoid arthritis, osteoarthritis and eye inflammation, cardio-vascular disorder, asthma, shock syndrome or other diseases mediated by the PAF, the compounds of Formula (I) may be administered orally, topically, parenterally, by inhalation spray or rectally in dosage unit formulations containing conventional non-toxic pharmaceutically acceptable carriers, adjuvants and vehicles. The term parenteral as used herein includes subcutaneous injections, intravenous, intramuscular, intrasternal injection or infusion techniques. In addition to the treatment of warm-blooded animals such as mice, rats, horses, cattle, sheep, dogs, cats, etc., the compounds of the invention are effective in the treatment of humans.

The pharmaceutical compositions containing the active ingredient may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may

be employed. They may also be coated by the techniques described in the U.S. Patents 4,256,108; 4,166,452; and 4,265,874 to form osmotic therapeutic tablets for control release.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin, or olive oil.

Aqueous suspensions contain the active materials in admixture with excipients suitable for the manufacture of aqueous suspensions. Such excipients are suspending agents, for example sodium carboxymethylcellulose, methylcellulose, hydroxypropylmethylcellulose, sodium alginate, polyvinylpyrrolidone, gum tragacanth and gum acacia; dispersing or wetting agents may be a naturally-occuring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl, p-hydroxybenzoate, one or more coloring agents, one or more flavoring agents, and one or more sweetening agents, such as sucrose or saccharin.

Oily suspensions may be formulated by suspending the active ingredients in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present.

The pharmaceutical compositions of the invention may also be in the form of oil-in-water emulsions. The oily phase may be a vegetable oil, for example olive oil or arachis oil, or a mineral oil, for example liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening and flavoring agents.

Syrups and elixirs may be formulated with sweetening agents, for example glycerol, propylene glycol, sorbitol or sucrose. Such formulations may also contain a demulcent, a preservative and flavoring and coloring agents. The pharmaceutical compositions may be in the form of a sterile injectable aqueous or oleagenous suspension. This suspension may be formulated according to the known art using those suitable dispersing or wetting agents and suspending agents which have been mentioned above. The sterile injectable preparation may also be sterile injectable solution or suspension in a non-toxic parenterally-acceptable diluent or solvent, for example as a solution in 1,3-butane diol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

The compounds of Formula (I) may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

For topical use, creams, ointments, jellies, solutions or suspensions, etc., containing the compounds of Formula (I) are employed.

Dosage levels of the order of from about 0.1 mg to about 140 mg per kilogram of body weight per day are useful in the treatment of the above-indicated conditions (about 0.5 mg to about 7 gms. per patient per day). For example, inflammation may be effectively treated by the administration of from about 0.01 to 50 mg of the compound per kilogram of body weight per day (about 1.0 mg to about 3.5 gms per patient per day).

The amount of active ingredient that may be combined with the carrier materials to produce a single dosage form will vary depending upon the host treated and the particular mode of administration. For example, a formulation intended for the oral administration of humans may contain from 0.5 mg to 5 gm of active agent compounded with an appropriate and convenient amount of carrier material which may vary from about 5 to about 95 percent of the total composition. Dosage unit forms will generally contain between from about 1 mg to about 500 mg of an active ingredient.

It will be understood, however, that the specific dose level for any particular patient will depend upon a variety of factors including the activity of the specific compound employed, the age, body weight, general health, sex, diet, time of administration, route of administration, rate of excretion, drug combination and the severity of the particular disease undergoing therapy.

D. Bioassay Results Supporting the utility of the compounds of the present invention

It has been found that the compounds of formula (I) exhibit antagonistic activities with respect to PAF. They inhibit *in vitro* PAF-induced functions in both the cellular and tissue levels by changing the PAF binding to its specific receptor site. This ability to inhibit the PAF binding to its specific receptor binding site on rabbit platelet plasma membranes was measured by an assay recently developed by us.

The inhibition of $H^3$-PAF binding to the rabbit platelet plasma membrane by a PAF antagonist of Formula (I) was determined by isotopic labeling and filtration techniques. Generally, a series of Tris-buffered solutions of the selected antagonist at predetermined concentrations were prepared. Each of these solutions contains 1 pmole of $^3$H-PAF, a known amount of the test antagonist, and a sufficient amount of the pH 7.5 Tris-buffer solution (10mM Tris, 0.25% bovine serum albumin, and 150 mM NaCl per ml water) to make the final volume of 1 ml. After adding into a set of test tubes each with a 100 $\mu$g of the platelet plasma membrane suspension (S.B. Hwang, et al., Biochemistry, Vol. 22, pp. 4756-4763, 1983) and one of the Tris-buffer solutions described above, the resulting mixture in each test tube was incubated at 0 °C for about one hour or until the reaction was complete. Two control samples, one of which ($C_1$) contains all the ingredients described above except the antagonist and the other ($C_2$) contains $C_1$ plus a 1000-fold excess of unlabeled PAF, were also prepared and incubated simultaneously with the test samples. After the incubation was completed, the contents of each test tube were filtered under *vacuo* through a Whatman GF/C fiberglass filter and the residue washed rapidly several times with a total of 20 ml cold (0°-5°C) Tris-buffer solution. Each washed residue was then suspended in 10 ml scintillation solution (Aquasol 2, New England Nuclear, Connecticut) and the radioactivity was counted in a Packard Tri-Carb 460CD Liquid Scintillation System. Defining the counts from a test sample as "Total binding with antagonist"; the counts from the control sample $C_1$, as "Total binding $C_1$"; and the counts from the control sample $C_2$ as "non-specific binding $C_2$", the percent inhibition of each test antagonist can be determined by the following equation:

$$\text{\% Inhibition} = \frac{(\text{Total binding } C_1) - \left(\begin{array}{c}\text{Total binding}\\ \text{with antagonist}\end{array}\right)}{\text{Specific binding}} \times 100$$

$$\text{Specific binding} = (\text{Total binding } C_1) - (\text{non-specific binding } C_2)$$

The antagonistic activity of the compounds of structural formula (I) is illustrated in the following tables (A) & (B):

## Table (A)

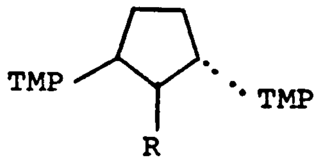

| R | PAF Receptor Binding Inhibition $IC_{50}$(nM)(rabbit) | Percent Inhibition |
|---|---|---|
| 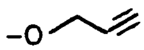 | 1.0 | |
| 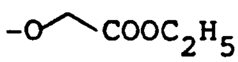 | 3.6 | |
| 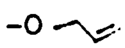 | 4.8 | |
| $=NOCH_3$ | 5.0 | |
| =NOH | 5.8 | |
| $-OCH_3$ | 8.3 | |
| 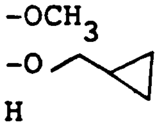 | 19 | |
| H | 20 | |
| 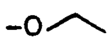 | 30 | |
| 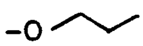 | 30 | |
| $-O-CH_2C_6H_5$ | 48 | |
| $-O-CO-CH_3$ | 100 | |
| 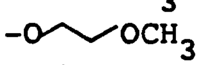 | 100 | |
| 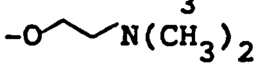 | 100 | |
| -OH | | 84% at 500 nM |
| 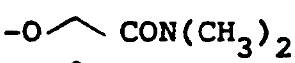 | | 23% at 300 nM |
| 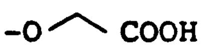 | | 16% at 300 nM |

## Table (B)

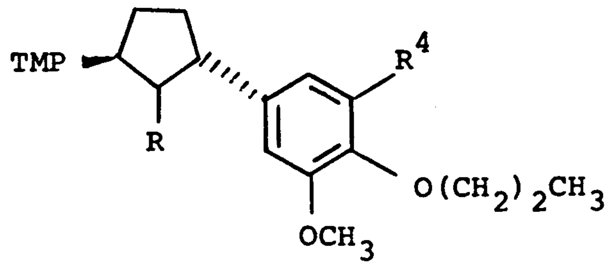

| R | $R^4$ | PAF Receptor Binding Inhibition $IC_{50}$(nM)(rabbit) | Percent Inhibition |
|---|---|---|---|
| $-OCH_3$ | $SO_2CH_3$ | 4 | |
| $-OCH_3$ | $SOCH_3$ | 20 | |
| $-OCH_3$ | $SCH_3$ | 15 | |
| $-OH$ | $SCH_3$ | | 23% at 300 nM |
| ⋯ $OCH_3$ | $SO_2CH_3$ | 3 | |
| ⋯ $OCH_3$ | $SOCH_3$ | 10 | |
| ⋯ $OCH_3$ | $SCH_3$ | 3 | |
| ⋯ $OH$ | $SCH_3$ | | 38% at 300 nM |
| $-OCH_3$ | Br | 50 | |
| $-OCH_3$ | CN | 160 | |
| ⋯ $OCH_3$ | CN | 25 | |
| $-OCH_3$ | $(CO)NH_2$ | 200 | |
| ⋯ $OCH_3$ | $(CO)NH_2$ | 100 | |

## Claims

**1.** A compound of formula:

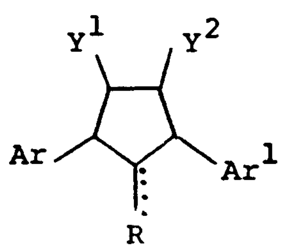

wherein

R is (a) when R is connected to the cyclopentane ring by a single bond

(1) $-(X)_m(CH_2)_nR^1$ wherein X is O, $NR^2$, $N^+R^2R^3$, S, SO, $SO_2$, $SO_3$ or CO; m is 0 or 1; n

is 1-6; $R^1$, $R^2$, $R^3$ are as defined below;

(2) $-X^1R^{10}$ wherein $X^1$ is O, S, $NR^2$ or $N^+R^2R^3$ and $R^{10}$ is $R^1$ except that it cannot be definitions (h)-(r) of $R^1$;

(3) $-X^2R^2$ wherein $X^2$ is SO, $SO_2$, $SO_3$, $SO_2NR^3$, $NR^3$(CO), $NR^3$(CO)NH, or $NHSO_2$; or

(4) $R^1$;

(b) when R is connected to the cyclopentane ring by a double bond:

(1) $=C-R^2$;

(2) =O; or

(3) $=NOR^2$;

$R^1$ is (a) H;

(b) loweralkyl;

(c) loweralkenyl;

(d) lowercycloalkyl ;

(e) loweralkynyl;

(f) $-(CO)NR^2R^3$ where $R^2$ and $R^3$ independently are H, $C_{1-8}$ alkyl, $C_{3-8}$ cycloalkyl or $C_{1-4}$ perhaloalkyl;

(g) $-(CO)R^2$;

(h) $-S(O)_qR^2$ where q represents 0-2;

(i) $-O(CO)R^2$;

(j) $-SO_2NR^2R^3$;

(k) $-NR^2(CO)R^3$;

(l) $-NR^2(CO)NHR^3$;

(m) $-NHSO_2R^2$;

(n) $-OR^2$;

(o) $-NR^2R^3$;

(p) -CN;

(q) $-NO_2$;

(r) halo;

(s) perhaloloweralkyl;

(t) phenyl or substituted phenyl as defined below; or

(u) $-(CO)OR^2$;

Ar and $Ar^1$ independently are:

(a) phenyl or substituted phenyl of formula

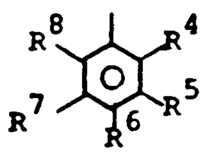

where $R^4$-$R^8$ independently represent H; $R^2$; YO- wherein Y is $R^2$, loweralkenyl, loweralkynyl,

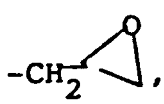,

$-CH_2C(O)OR^2$, $-CH_2OR^2$, $-CH_2C_{3-8}$cycloalkyl, $-CH_2Ar^2$ wherein $Ar^2$ is phenyl or substituted phenyl, $-CH_2-CH(OH)-CH_2OH$; $R^2S$-; $R^2SO$-; $R^2SO_2$-; $CF_3O$-; $CF_3S$; $CF_3SO$; $CF_3SO_2$; $CH_3OCH_2O$-; $R^2R^3N$-; $-OCH_2CO_2R^2$ ; $SO_2NR^2R^3$; $-CO_2R_2$; $CONR^2R^3$; $-NR^2COR^3$; $-OCONH_2$; $-CR^2R^3R^9$ wherein $R^9$ is the same as or different from $R^2$; $-CH_2OR^2$; $-CH_2CO_2R^2$; $-CH_2OCOR^3$; $-CH_2O-CO-OR^2$; $-NHCH_2COOR^2$; halo; $-N^+R^2R^3R^9$ $W^-$ wherein W is an anion; $-NR^2SO_2R^3$; $-COR^2$; $NO_2$; CN; or $R^4$-$R^5$, $R^5$-$R^6$, $R^6$-$R^7$ and $R^7$-$R^8$ joined together and form a ring of formula $-OCH_2O-$, $-OCH_2CH_2O-$, or $-OCH_2CH_2N-$;

(b) monoheteroaryl, di- or polyheteroaryl or fused hetroaryl containing 1 to 3 of any one or more of the heteroatoms N, S or O;

with the proviso that Ar and $Ar^1$ cannot be simultaneously phenyl when R is -OH; =O; or =NOH; and $Y^1$ and $Y^2$ independently are H, $C_{1-6}$ alkyl, C1, F, $C_{1-6}$ haloalkyl.

2. The compound of Claim 1 wherein the compound is of formula

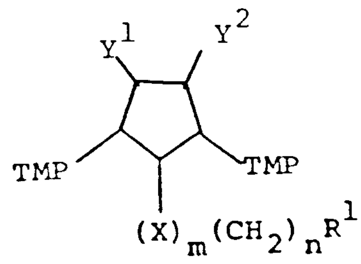

or an isomer thereof wherein TMP is 3,4,5-trimethoxyphenyl, X is O or S; m is 0-1; n is 1-2; and $R^1$, $Y^1$ and $Y^2$ are as previously defined.

3. The compound of Claim 1 wherein the compound is of formula

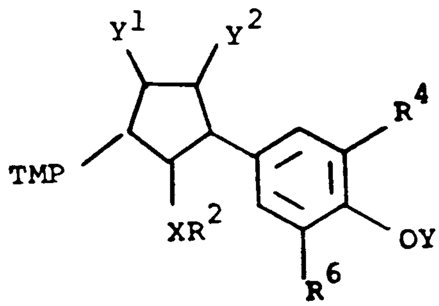

or an isomer thereof wherein TMP, $R^2$, $R^4$, $R^6$, Y, $Y^1$ and $Y^2$ are as previously defined; X is O or S.

4. The compound of Claim 1 wherein the compound is of formula

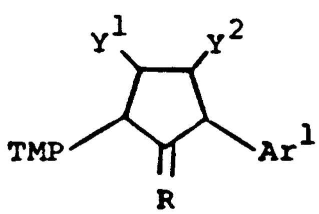

wherein TMP is as previously defined, R is $NOR^2$ or O; and $Ar^1$ is TMP or a radical of formula

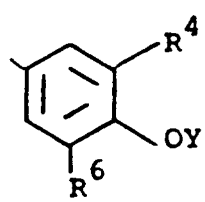

where $R^4$, Y, $Y^1$, $Y^2$ and $R^6$ are as previously defined.

5. The compound of Claim 1 which is of formula

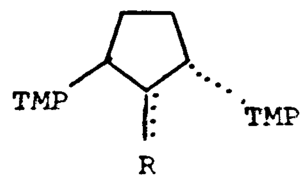

wherein TMP is 3,4,5-trimethoxyphenyl;

R is
(a) 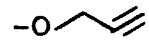
(b) 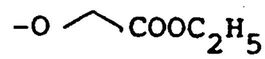
(c) 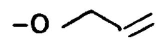
(d) $=NOCH_3$
(e) $=NOH$
(f) $-OCH_3$
(g) 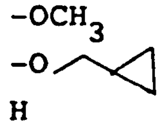
(h) H
(i) 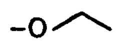
(j) 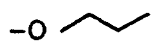
(k) $-O-CH_2C_6H_5$
(l) $-O-CO-CH_3$
(m) 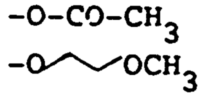
(n) 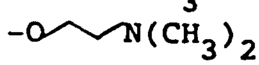
(o) $-OH$
(p) 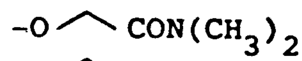
(q) 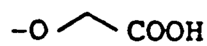

6. The compound of Claim 1 which is of formula

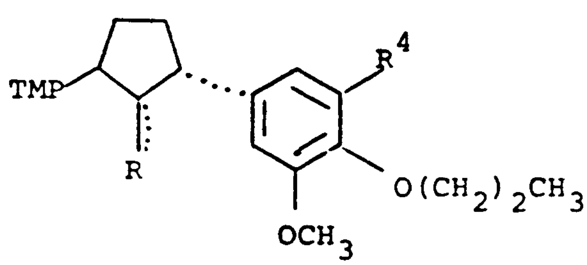

or an isomer thereof wherein TMP is 3,4,5-trimethoxyphenyl;

R is (a) OH; or
(b) $OCH_3$; and

$R^4$ is (a) $SO_2CH_3$;
(b) $SOCH_3$;
(c) $SCH_3$
(d) Br;

(e) CN; or
(f) $(CO)NH_2$.

**7.** The compound of Claim 1 which is

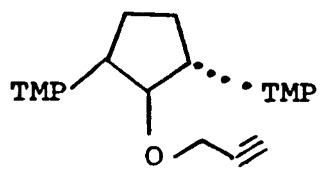

or an isomer thereof.

**8.** A pharmaceutical composition for treating a disease or a disorder mediated by PAF comprising a pharmaceutical carrier and a therapeutically effective amount of a compound as claimed in any one of claims 1 to 7.

**9.** The use of a compound as claimed in any one of claims 1 to 7 for the preparation of a medicament useful for the treatment of a disease or a disorder mediated by PAF.

**10.** A process for preparing the compound as claimed in Claim 1; comprising
(a) tosylating a compound of formula II:

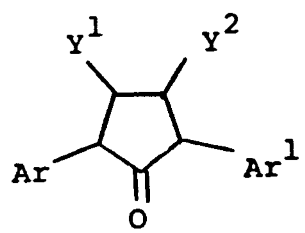

(II)

or an isomer thereof and treating the resultant tosylhydrazone derivative with a reducing agent;
(b) Reducing a compound of formula II to a cyclopentanol of formula

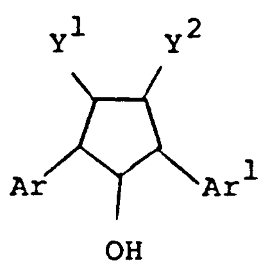

or an isomer thereof followed by alkylating the cyclopentanol with a compound of formula $R^{10}X$ wherein $R^{10}$ is $R^2$ as previously defined; $-CH_2COOR°$ wherein R° is $C_{1-6}$alkyl; $-CH_2-C\equiv CH$; $-CH_2CH=CH_2$; $-CH_2$-phenyl; $-CH_2$-substituted phenyl; $-CH_2OC_{1-6}$alkyl; $-(CH_2)_2NC_{1-6}$-alkyl; $-CH_2-CONR^2R^3$; and X is halo;
(c) treating a compound of formula:

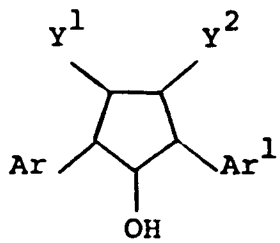

with an anhydride of formula ($(R^2CO_2)O$) or an acyl halide of formula $R^2(CO)X$; or
(d) treating a compound of formula II with $NH_2OR^2$.

**11.** The process of Claim 10 for the preparation of a compound of formula

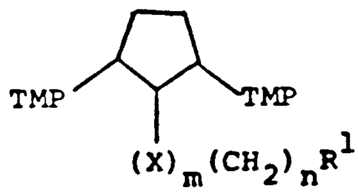

or an isomer thereof wherein TMP is 3,4,5-trimethoxyphenyl, X is O or S; m is 0-1; n is 1-2; and $R^1$ is as previously defined.

**Revendications**

**1.** Composé de formule :

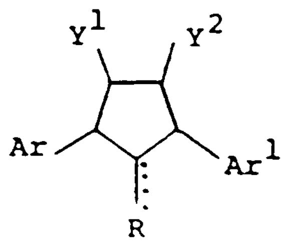

dans laquelle

R est (a) lorsque R est lié au noyau cyclopentane par une simple liaison :

(1) $-(X)m(CH_2)_nR^1$, où X est O, $NR^2$, $N^+R^2R^3$, S, SO, $SO_2$, $SO_3$ ou CO; m est nul ou égal à 1; n a une valeur de 1-6; $R^1$, $R^2$, $R^3$ sont tels que définis ci-dessous;

(2) $-X^1R^{10}$, où $X^1$ est O, S, $NR^2$ ou $N^+R^2R^3$ et $R^{10}$ est $R^1$, sauf qu'il ne peut pas avoir les définitions (h)-(r) de $R^1$;

(3) $-X^2R^2$, où $X^2$ est SO, $SO_2$, $SO_3$, $SO_2NR^3$, $NR^3(CO)$, $NR^3(CO)NH$ ou $NHSO_2$; ou

(4) $R^1$;

(b) lorsque R est lié au noyau cyclopentane par une double liaison :

(1) $=C-R^2$;

(2) $=O$; ou

(3) $=NOR^2$;

$R^1$ est (a) H;

(b) un groupe alkyle inférieur;

(c) un groupe alcényle inférieur;

(d) un groupe cycloalkyle inférieur;

(e) un groupe alcynyle inférieur;

(f) $-(CO)NR^2R^3$, dans lequel $R^2$ et $R^3$ sont, indépendamment, H, des groupes alkyle en $C_1$-$C_8$, cycloalkyle en $C_3$-$C_8$ ou perhalogénoalkyle en $C_1$-$C_4$;

(g) $-(CO)R^2$;
(h) $-S(O)_qR^2$, dans lequel q représente 0-2:
(i) $-O(CO)R^2$;
(j) $-SO_2NR^2R^3$;
(k) $-NR^2(CO)R^3$;
(l) $-NR^2(CO)NHR^3$;
(m) $-NHSO_2R^2$;
(n) $-OR^2$;
(o) $-NR^2R^3$;
(p) -CN;
(q) $-NO_2$;
(r) halogéno;
(s) perhalogénoalkyle inférieur;
(t) phényle ou phényle substitué, tel que défini ci-dessous; ou
(u) $-(CO)OR^2$;

Ar et $Ar^1$ sont, indépendamment,
(a) des groupes phényle ou phényle substitués de formule

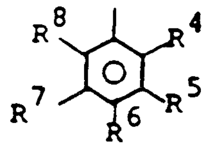

dans laquelle $R^4$-$R^8$ représentent, indépendamment, H; $R^2$; YO-, dans lequel Y est $R^2$, un groupe alcényle inférieur; alcynyle inférieur,

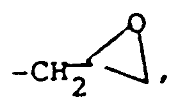

$-CH_2C(O)OR^2$, $-CH_2OR^2$, $-CH_2$-cycloalkyle en $C_3$-$C_8$, $-CH_2Ar^2$, où $Ar^2$ est un groupe phényle ou phényle substitué, $-CH_2-CH(OH)-CH_2OH$; $R^2S$-; $R^2SO$-; $R^2SO_2$-; $CF_3O$-; $CF_3S$; $CF_3SO$; $CF_3SO_2$; $CH_3OCH_2O$-; $R^2R^3N$-; $-OCH_2CO_2R^2$; $SO_2NR^2R^3$; $-CO_2R^2$; $-CONR^2R^3$; $-NR^2COR^3$; $-OCONH_2$; $-CR^2R^3R^9$ où $R^9$ est identique ou différent de $R^2$; $-CH_2OR^2$; $-CH_2CO_2R^2$; $-CH_2OCOR^3$; $-CH_2O-CO-OR^2$; $-NHCH_2COOR^2$; halogéno; $N^+R^2R^3R^9W^-$, où $W^-$ est un anion; $NR^2SO_2R^3$; $COR^2$; $NO_2$; CN; ou $R^4$-$R^5$, $R^5$-$R^6$, $R^6$-$R^7$ et $R^7$-$R^8$ sont liés l'un à l'autre et forment un noyau de formule $-OCH_2O$-, $-OCH_2CH_2O$- ou $-OCH_2CH_2N$-;
(b) des groupes monohétéroaryle, di- ou polyhétéroaryle, ou hétéroaryle condensé contenant de 1 à 3 d'un ou plusieurs des hétéroatomes N, S ou O;

à condition que Ar et $Ar^1$ ne soient pas simultanément des groupes phényle, où R est -OH, =O ou =NOH; et

$Y^1$ et $Y^2$ sont, indépendamment, H, des groupes alkyle en $C_1$-$C_6$, Cl, F, halogénoalkyle en $C_1$-$C_6$.

**2.** Composé selon la revendication 1, dans lequel le composé a pour formule

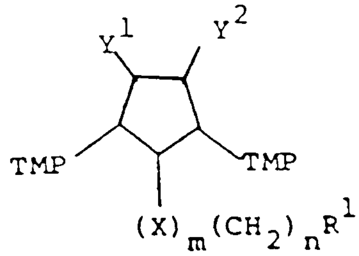

ou un de ses isomères, où TMP est un groupe 3,4,5-triméthoxyphényle, X est O ou S; m est égal à 0-1; n est égal à 1-2; et $R^1$, $Y^1$ et $Y^2$ sont tels que définis ci-dessus.

**3.** Composé selon la revendication 1, dans lequel le composé a pour formule

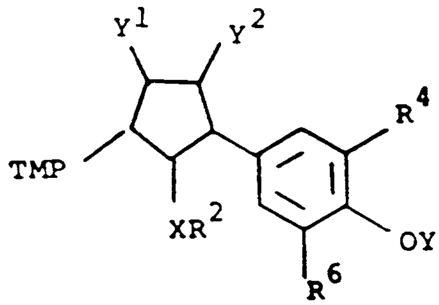

ou un de ses isomères, où TMP, $R^2$, $R^4$, $R^6$, Y, $Y^1$ et $Y^2$ sont tels que définis ci-dessus; X est O ou S.

**4.** Composé selon la revendication 1, dans lequel le composé a pour formule

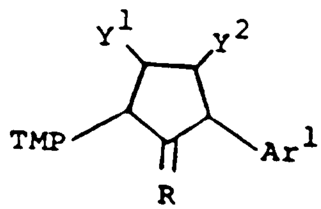

dans laquelle TMP est tel que défini ci-dessus, R est $NOR^2$ ou O; et $Ar^1$ est TMP ou un radical de formule

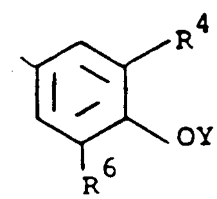

dans laquelle $R^4$, Y, $Y^1$, $Y^2$ et $R^6$ sont tels que définis ci-dessus.

**5.** Composé selon la revendication 1, qui a pour formule

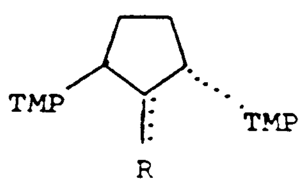

dans laquelle TMP est un groupe 3,4,5-triméthoxyphényle; R est

(a) 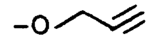

(b) 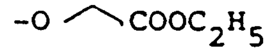

(c) 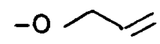

(d) $=NOCH_3$

(e) $=NOH$

(f) $-OCH_3$

(g) 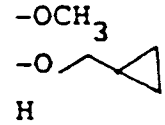

(h) H

(i) 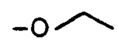

(j) 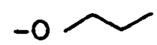

(k) $-O-CH_2C_6H_5$

(l) $-O-CO-CH_3$

(m) 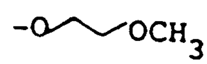

(n) 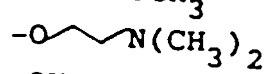

(o) $-OH$

(p) 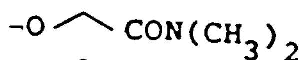

(q) 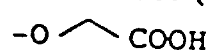

6. Composé selon la revendication 1, qui a pour formule

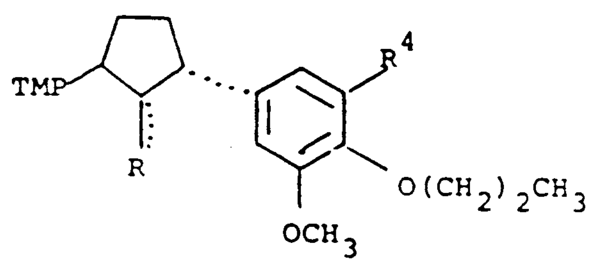

ou un de ses isomères, où TMP est un groupe 3,4,5-triméthoxyphényle;

R est (a) OH; ou
(b) $OCH_3$; et

$R^4$ est (a) $SO_2CH_3$;
(b) $SOCH_3$;
(c) $SCH_3$;
(d) Br;
(e) CN; ou
(f) $(CC)NH_2$.

7. Composé selon la revendication 1, qui est

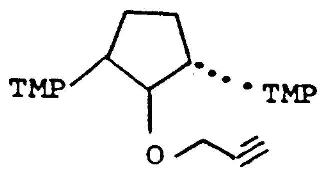

ou un de ses isomères.

8. Composition pharmaceutique servant à traiter une maladie ou une affection ayant pour médiateur le PAF, comprenant un véhicule pharmaceutique et une quantité thérapeutiquement efficace d'un composé selon l'une quelconque des revendications 1 à 7.

9. Utilisation d'un composé selon l'une quelconque des revendications 1 à 7 pour préparer un médicament utile pour le traitement d'une maladie ou d'une affection ayant pour médiateur le PAF.

10. Procédé de préparation du composé selon la revendication 1, comprenant
(a) la tosylation d'un composé de formule II :

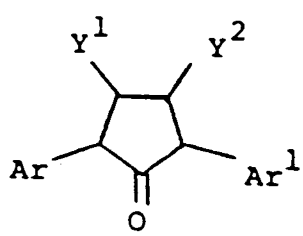

(II)

ou d'un de ses isomères, et le traitement du dérivé tosylhydrazone résultant avec un agent réducteur;
(b) la réduction d'un composé de formule II en un cyclopentanol de formule

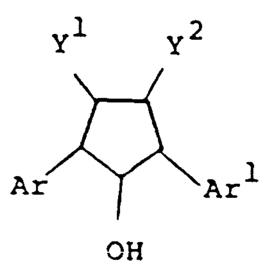

ou un de ses isomères, suivie de l'alkylation du cyclopentapol avec un composé de formule $R^{10}X$, dans laquelle $R^{10}$ est $R^2$ tel que défini ci-dessus; $-CH_2COOR°$, où R° est un groupe alkyle en $C_1$-$C_6$; $-CH_2-C{\equiv}CH$; $-CH_2CH=CH_2$; $-CH_2$-phényle; $-CH_2$-phényle substitué; $-CH_2O$-alkyle en $C_1$-$C_6$; $-(CH_2)_2N$-alkyle en $C_1$-$C_6$; ou $-CH_2CONR^2R^3$; et X est un halogène;
(c) le traitement d'un composé de formule :

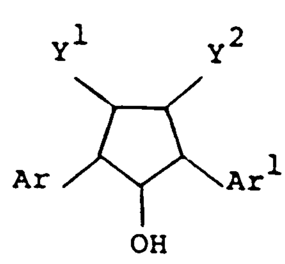

avec un anhydride de formule $(R^2CO_2)O$ ou un halogénure d'acyle de formule $R^2(CO)X$; ou
(d) le traitement d'un composé de formule II avec $NH_2OR^2$.

**11.** Procédé selon la revendication 10 pour préparer un composé de formule

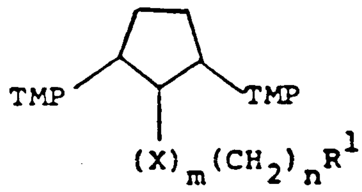

ou un de ses isomères, où TMP est un groupe 3,4,5-triméthoxyphényle, X est O ou S; m est égal à 0-1; n est égal à 1-2; et $R^1$ est tel que défini ci-dessus.

**Patentansprüche**

**1.** Verbindung der Formel

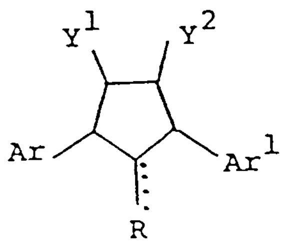

worin

R (a) wenn R durch eine Einfachbindung mit dem Cyclopentanring verbunden ist
(1) $-(X)_m(CH_2)_nR^1$, worin X O, $NR^2$, $N^+R^2R^3$, S, SO, $SO_2$, $SO_3$ oder CO ist; m 0 oder 1 ist; n 1 bis 6 ist; $R^1$, $R^2$, $R^3$ wie nachstehend definiert sind;
(2) $-X^1R^{10}$, worin $X^1$ O, S, $NR^2$ oder $N^+R^2R^3$ ist und $R^{10}$ $R^1$ ist, außer daß es nicht die Definitionen (h)-(r) von $R^1$ haben kann;
(3) $-x^2R^2$, worin $X^2$ SO, $SO_2$, $SO_3$, $SO_2NR^3$, $NR^3(CO)$, $NR^3(CO)NH$ oder $NHSO_2$ ist; oder
(4) $R^1$ ist;
(b) wenn R über eine Doppelbindung mit dem Cyclopentanring verbunden ist
(1) $=C-R^2$;
(2) $=O$; oder
(3) $=NOR^2$ ist;

$R^1$ (a) H;
(b) Niederalkyl;
(c) Niederalkenyl;
(d) Niedercycloalkyl;
(e) Niederalkinyl;
(f) $-(CO)NR^2R^3$, worin $R^2$ und $R^3$ unabhängig H, $C_{1-8}$-Alkyl, $C_{3-8}$-Cycloalkyl oder $C_{1-4}$-Perhalogenalkyl sind;
(g) $-(CO)R^2$;
(h) $-S(O)_qR^2$, worin q 0 bis 2 darstellt;
(i) $-O(CO)R^2$;
(j) $-SO_2NR^2R^3$;
(k) $-NR^2(CO)R^3$;
(l) $-NR^2(CO)NHR^3$;
(m) $-NHSO_2R^2$;
(n) $-OR^2$;
(o) $-NR^2R^3$;

(p) -CN;
(q) $-NO_2$;
(r) Halogen;
(s) Perhalogenniederalkyl;
(t) Phenyl oder substituiertes Phenyl, wie nachstehend definiert; oder
(u) $-(CO)OR^2$ ist;

Ar und $Ar^1$ unabhängig

(a) Phenyl oder substituiertes Phenyl der Formel

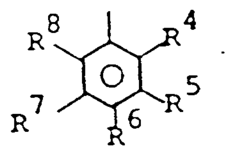

,

worin $R^4$-$R^8$ unabhängig H; $R^2$; YO-, worin Y $R^2$ , Niederalkenyl, Niederalkinyl,

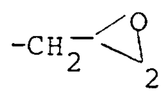

,

$-CH_2C(O)OR^2$, $-CH_2OR^2$, $-CH_2C_{3-8}$-Cycloalkyl, $-CH_2Ar^2$ , worin $Ar^2$ Phenyl oder substituiertes Phenyl ist, $-CH_2-CH(OH)-CH_2OH$ ist; $R^2S$-; $R^2SO$-; $R^2SO_2$-; $CF_2O$-; $CF_3S$; $CF_3SO$; $CF_3SO_2$; $CH_3OCH_2O$-; $R^2R^3N$-; $-OCH_2CO_2R^2$; $SO_2NR^2R^3$; $-CO_2R^2$ ; $-CONR^2R^3$; $-NR^2COR^3$; $-OCONH_2$; $-CR^2R^3R^9$, worin $R^9$ gleich oder verschieden von $R^2$ ist; $-CH_2OR^2$; $-CH_2CO_2R^2$; $-CH_2OCOR^3$; $-CH_2O-CO-OR^2$; $-NHCH_2COOR^2$; Halogen; $-N^+R^2R^3R^9W^-$, worin $W^-$ ein Anion ist; $-NR^2SO_2R^3$; $-COR^2$; $NO_2$; CN darstellen oder $R^4$-$R^5$, $R^5$-$R^6$, $R^6$-$R^7$ und $R^7$-$R^8$ zusammengenommen einen Ring der Formel $-OCH_2O-$, $-OCH_2CH_2O-$ oder $-OCH_2CH_2N-$ bilden;

(b) Monoheteroaryl, Di- oder Polyheteroaryl oder kondensiertes Heteroaryl mit 1 bis 3 eines oder mehrerer der Heteroatome N, S oder O sind;

mit der Maßgabe, daß-Ar und $Ar^1$ nicht gleichzeitig Phenyl sein können, wenn R -OH, =O oder =NOH ist; und $Y^1$ und $Y^2$ unabhängig H, $C_{1-6}$-Alkyl, Cl, F, $C_{1-6}$-Halogenalkyl sind.

2. Verbindung nach Anspruch 1, worin die Verbindung eine der Formel ist:

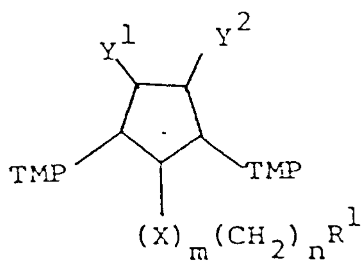

oder ein Isomer davon, worin TMP 3,4,5-Trimethoxyphenvl ist, X O oder S ist, m 0 bis 1 ist, n 1 bis 2 ist und $R^1$, $Y^1$ und $Y^2$ wie vorstehend definiert sind.

3. Verbindung nach Anspruch 1, worin die Verbindung eine der Formel ist

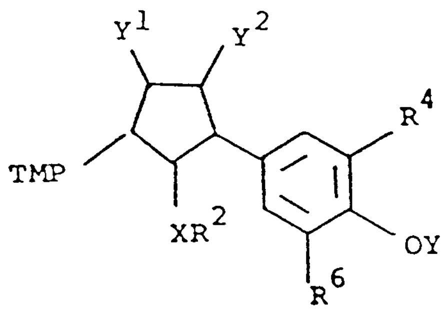

oder ein Isomer davon, worin TMP, $R^2$ $R^4$, $R^6$, Y, $Y^1$ und $Y^2$ wie vorstehend definiert sind und X O oder S ist.

**4.** Verbindung nach Anspruch 1, worin die Verbindung eine der Formel ist

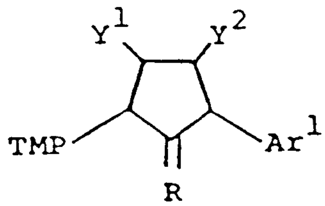

worin TMP wie vorstehend definiert ist, R $NOR^2$ oder O ist und $Ar^1$ TMP oder ein Rest der Formel ist:

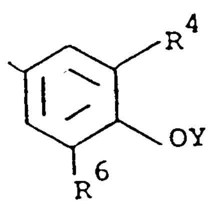

worin $R^4$, Y, $Y^1$, $Y^2$ und $R^6$ wie vorstehend definiert sind.

**5.** Verbindung nach Anspruch 1 der Formel:

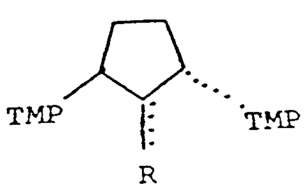

worin TMP 3,4,5-Trimethoxyphenyl ist und

R
(a) 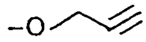
(b) 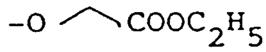
(c) 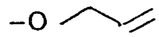
(d) $=NOCH_3$
(e) $=NOH$
(f) $-OCH_3$
(g) 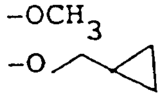
(h) H
(i) 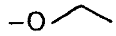
(j) 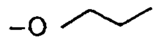
(k) $-O-CH_2C_6H_5$
(l) $-O-CO-CH_3$
(m) 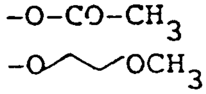
(n) 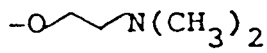
(o) $-OH$
(p) 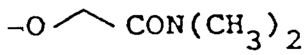
(q) 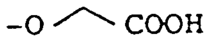 .

6. Verbindung nach Anspruch 1 der Formel

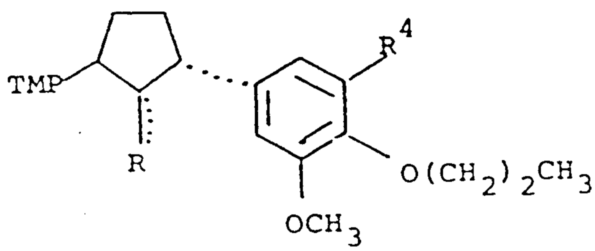

oder ein Isomer davon, worin TMP 3,4,5-Trimethoxyphenyl ist;

R (a) OH oder
(b) $OCH_3$ ist und

$R^4$ (a) $SO_2CH_3$,
(b) $SOCH_3$,
(c) $SCH_3$,
(d) Br,
(e) CN oder
(f) $(CO)NH_2$ ist.

7. Verbindung nach Anspruch 1, welche

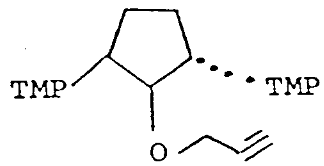

oder ein Isomer davon ist.

8. Pharmazeutische Zusammensetzung zur Behandlung einer durch PAF vermittelten Krankheit oder Störung, welche einen pharmazeutischen Träger und eine therapeutisch wirksame Menge einer Verbindung nach einem der Ansprüche 1 bis 7 umfaßt.

9. Verwendung einer Verbindung nach einem der Ansprüche 1 bis 7 zur Herstellung eines Medikaments, das zur Behandlung einer von PAF vermittelten Krankheit oder Störung geeignet ist.

10. Verfahren zur Herstellung einer Verbindung nach Anspruch 1 durch
(a) Tosylieren einer Verbindung der Formel II:

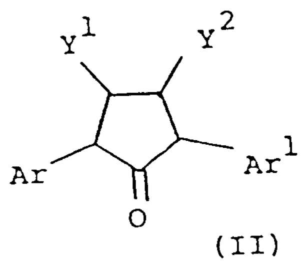

(II)

oder eines Isomers davon und Behandeln des resultierenden Tosylhydrazonderivats mit einem Reduktionsmittel;
(b) Reduzieren einer Verbindung der Formel II zu einem Cyclopentanol der Formel

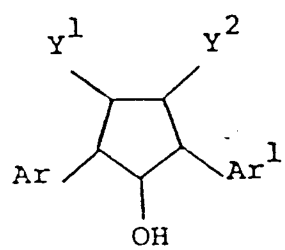

oder einem Isomer davon, gefolgt von der Alkylierung des Cyclopentanols mit einer Verbindung der Formel $R^{10}X$, worin $R^{10}$ $R^2$ ist, wie vorstehend definiert, $-CH_2COOR°$, worin R° $C_{1-6}$-Alkyl, $-CH_2-C{\equiv}CH$, $-CH_2CH=CH_2$, $-CH_2$-Phenyl, $-CH_2$-substituiertes Phenyl, $-CH_2OC_{1-6}$-Alkyl, $-(CH_2)_2NC_{1-6}$-Alkyl oder $-CH_2-CONR^2R^3$ ist und X Halogen ist;
(c) Behandeln einer Verbindung der Formel

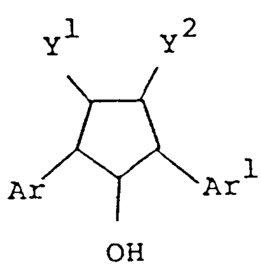

mit einem Anhydrid der Formel ($R^2CO_2$)O oder einem Acylhalogenid der Formel $R^2$(CO)X; oder
(d) Behandeln einer Verbindung der Formel II mit $NH_2OR^2$.

**11.** Verfahren nach Anspruch 10 zur Herstellung einer Verbindung der Formel

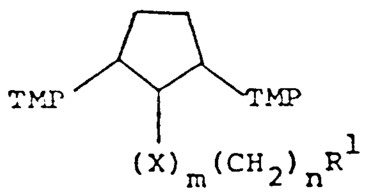

oder eines Isomers davon, worin TMP 3,4,5-Trimethoxyphenyl ist, X O oder S ist, m 0-1 ist, n 1-2 ist und $R^1$ wie vorstehend definiert ist.